# EUROPEAN PATENT SPECIFICATION

(11) **EP 3 177 737 B1**
(45) Date of publication and mention of the grant of the patent: **14.10.2020**
(21) Application number: 15807518.4
(22) Date of filing: 10.06.2015
(51) Int. Cl.: C12Q 1/68, C07K 14/68

(54) **NOVEL MODULATORS OF MELANOCORTIN RECEPTORS**
NEUARTIGE MODULATOREN VON MELANOCORTIN-REZEPTOREN
NOUVEAUX MODULATEURS DES RÉCEPTEURS DE LA MÉLANOCORTINE

(30) Priority: 10.06.2014 US 201414300991; 25.06.2014 US 201462017137 P
(43) Date of publication of application: 14.06.2017
(73) Proprietor: Arizona Board of Regents on behalf of the University of Arizona, Tucson, AZ 85721 (US)
(72) Inventor: CAI, Minying, Tucson, AZ 85721 (US); HRUBY, Victor, J., Tucson, AZ 85721 (US)
(74) Representative: Held, Stephan
(86) International application number: PCT/US2015/035180
(87) International publication number: WO 2015/191765

(56) References cited:
- US-A- 5 731 408
- VICTOR J. HRUBY ET AL: "Design of Peptide and Peptidomimetic Ligands with Novel Pharmacological Activity Profiles", ANNUAL REVIEW OF PHARMACOLOGY AND TOXICOLOGY., vol. 53, no. 1, 6 January 2013 (2013-01-06), pages 557-580, XP055389581, US ISSN: 0362-1642, DOI: 10.1146/annurev-pharmtox-010510-100456
- LUCAS DOEDENS ET AL: "Multiple N -Methylation of MT-II Backbone Amide Bonds Leads to Melanocortin Receptor Subtype hMC1R Selectivity: Pharmacological and Conformational Studies", JOURNAL OF THE AMERICAN CHEMICAL SOCIETY, vol. 132, no. 23, 16 June 2010 (2010-06-16), pages 8115-8128, XP055389484, US ISSN: 0002-7863, DOI: 10.1021/ja101428m
- Yaniv Linde: "Structure-activity relationship and metabolic stability studies of backbone cyclization and N-methylation of melanocortin peptides - Linde - 2008 - Peptide Science - Wiley Online Library", , 24 July 2008 (2008-07-24), XP055389491, Retrieved from the Internet: URL:http://onlinelibrary.wiley.com/doi/10. 1002/bip.21057/full [retrieved on 2017-07-10]
- CHUL-JIN LEE ET AL: "Solution structures and molecular interactions of selective melanocortin receptor antagonists", MOLECULES AND CELLS, vol. 30, no. 6, 23 November 2010 (2010-11-23), pages 551-556, XP055123000, ISSN: 1016-8478, DOI: 10.1007/s10059-010-0152-6

## Description

### CROSS REFERENCE

This application claims priority to U.S. Provisional Patent Application No. 62/017,137 filed June 25, 2014, and claims priority to U.S. Patent Application No. 14/300,991 filed on June 10, 2014.

### GOVERNMENT SUPPORT

This invention was made with the government support under Grant Nos. R01 DK017420, R01 GM108040, and P01 DA006284 awarded by NIH. The government has certain rights in the invention.

### FIELD OF THE INVENTION

The present invention relates to a series of N-methylated variations on a cyclic peptide, in particular to a series of N-methylated variations on the SHU9119 peptide. Methyl groups are added to a variety of places on a parmacophore of a base compound, and the agonizing and antagonizing properties of the resulting compounds are examined.

### BACKGROUND OF THE INVENTION

Melanocortin receptors, MC1-5R are a family of five receptor compounds that have varying effects related to melanin. All, but melanocortin-2, is capable of being activated by different types of melanocyte stimulating hormone (Yang). Prior to the invention, it has been difficult to target the receptors, except for MC2R, independently of one another. The key difference between this compound and similar compounds is that the present invention is specific to individual types of melanocortin receptors (Grimes, et. al). Each methylated form of the invention grants a different effect to certain melanocortin receptors, either agonizing them or antagonizing them. This would potentially reduce the amount of side effects incurred by the treatments involving the present invention.

The melanocortin system¹⁻³ remains a challenging target for rational peptide and peptidomimetic design as the 3D-topographical requirements for specific melanocortin receptor subtype recognition have not been fully elucidated.⁴⁻⁷ Nevertheless, the numerous multifaceted physiological functions of the five known subtypes of human melanocortin receptors (hMC1-5R), including skin pigmentation,^{8,9} control of the immune system,^{10,11} erectile function,^{12,13} blood pressure and heart rate,^{14,15} control of feeding behavior and energy homeostasis,^{3,6,16-21} modulation of aggressive/defensive behavior,^{22,23} and mediation of pain,²⁴⁻²⁶ continue to provide a strong stimulus for further development of potent and selective melanocortin agonists and antagonists.

Until recently, much of this work was focused on the hMC4R due to its direct involvement in the regulation of feeding behavior and energy homeostasis,^{3,6,16-21} as well as sexual behavior.^{1,6,12,13,27} The hMC3 receptor has been suggested to play a complementary role in weight control.^{20,21,28} Although the full scope of physiological functions of this receptor is yet to be unraveled, the current understanding based on the observed stimulation of food intake by peripheral administration of an MC3R-selective agonist²⁹ and MC3R agonist-induced inhibition of spontaneous action of Pro-opiomelanocortin (POMC) neurons³⁰ suggests that hMC3R is an inhibitory autoreceptor on POMC neurons.³ In addition, development of selective ligands for the hMC1 and hMC5 receptors is receiving increasing attention lately due to the roles of these receptors in regulation of pain, skin pigmentation,^{6,7} control of the immune system (hMC1R)^{8,11} and; in regulating exocrine gland function³¹ and coordinating central and peripheral signals for aggression (hMC5R).^{22,23}

On the other hand, development of selective ligands to melanocortin system bears intrinsic challenges due to conserved amino acid sequences and their structural similarity contained in the 7 transmembrane GPCR fold.^{1-3,32,33} Unlike other protein targets, hMCRs, known to be the smallest GPCRs have separate natural agonist and antagonist molecules for functional regulation.^{1-3,32,33} This aspect of hMCRs imposes a second dimension on melanocortin ligands for achieving selectivity not only to receptor subtype but also between the required agonistic and antagonistic properties. Designing such molecules which possess both functional selectivity and hMCR subtype selectivity from a four residue hMCR recognition sequence^{34,35} His-Phe-Arg-Trp is very demanding and necessitates to experiment with every possible molecule designing tool in peptide chemistry.

Great efforts have been made in the last decade to develop selective melanotropin peptides by following various general approaches,³⁶⁻³⁹ which include (a) D-amino acid scan/unnatural amino acid substitutions in linear α-, β- and γ-melanocortin stimulating hormone (MSH)-derived sequences,^{40,41} (b) hybridizations of the native MSH sequences with each other and with sequences of other bioactive peptides,⁴²⁻⁴⁴ (c) implementation of various global and local conformational constraints *via* peptide cyclization and employment of constrained amino acids,⁴⁵⁻⁵² (d) manipulation of steric factors that influence receptor-ligand interaction,^{49,53-55} (e) construction of small molecules based on β-turn peptidomimetics and "privileged structure" scaffolds, ⁵⁵⁻⁵⁹ and (f) multiple *N*-methylation.⁶⁰

Application of several of these strategies has resulted in the development of two cyclic peptides, i. Ac-Nle⁴-c[Asp⁵, D-Nal (2')⁷, Lys¹⁰]α-MSH(4-10)-NH₂ (SHU9119, D-Nal (2')⁷ henceforth is abbreviated as nal⁷),⁴⁸ a potent agonist for the hMC1R and hMC5R and a potent antagonist for the hMC3R and hMC4R; ii. Ac-Nle⁴-c[Asp⁵, D-Phe⁷, Lys¹⁰]α-MSH(4-10)-NH₂ (MT-II), a universal agonist for all the hMCRs.⁴⁶ The two ligands, although lacking exclusive receptor subtype selectivity, have been extensively used in understanding the combined functional aspects of hMCRs. The complexity of the melanocortin system can be envisaged from the amino acid sequences of SHU9119 and MT-II. Although these two ligands differ in side chain of a single amino acid (nal⁷ Vs. D-Phe⁷ in SHU9119 and MT-II, respectively) and possess a structural similarity,^{61,62} they give rise to entirely contrasting functional responses with melanocortins. The distinctness in activity of these two similar ligands has been attributed mainly to the differences in the disposition of functional side chains around the peptide backbone.

Noticeable variations in the activity of SHU9119 have been reported with any changes even in the chemical constitution of nal⁷ aromatic side chain, signifying the sensitivity of hMCRs towards their ligands.⁴⁸ Several amino acid substitution studies have been carried out on MT-II and SHU9119 to modify the structure.^{36,63} Replacement of His⁶ in SHU9119 with conformationally constrained amino acids resulted in selective agonists for hMC5R and antagonists for hMC3R and hMC4R.^{64,65} This lead to the understanding that the conformational restriction at His⁶ and around nal⁷ may help in receptor discrimination. Among the other studies carried out, it has also been shown that the activity of melanocortin ligands is sensitive to the side chain χ conformation.⁶⁶ Such outcomes suggest melanocortin ligand designs in which side chain disposition is altered while retaining the functional moieties unchanged. Furthermore, a proper insight into the structure driven responses of melanocortin subtypes requires a systematic perturbation of ligand conformation.

To accomplish this, the *N*-methylation strategy⁶⁷⁻⁶⁹ is applied to the melanocortin ligands. Recently, *N*-methylation of backbone amide NHs of peptides has been shown to substantially improve the physicochemical, structural and biological features of peptides.⁶⁷⁻⁷³ *N*-methylation stands as the simplest way to include conformational restraints into the peptide backbone as it introduces steric restrictions, allows for *cis*-peptide bonds, and prevents hydrogen bond formation. As these effects make *N*-methylated amino acids often act as turn promoting moieties (akin to prolines),^{67,73} the strategy helps in generating secondary structural features in peptides without changing the constituent peptide sequence. The specific impact of *N*-methylation on melanocortin peptide ligands has not been investigated systematically until this strategy is used on MT-II to develop completely selective hMC1R agonists.⁶⁰ Interestingly, in contrast to the parent MT-II, some of the *N-*methylated MT-II peptides possessed antagonistic activity, meaning a switch in functional selectivity while the amino acid sequence remained unchanged.

Based on these intriguing aspects and being deficient of melanocortin ligands exhibiting improved biological activity and pharmacokinetic profiles, the present invention is considered to modulate the peptide conformation and the functional side chain disposition of SHU9119 peptide by *N*-methylation of the backbone amide NHs. In search of a more selective melanocortin peptides and also to gain further insight into the intricate correlations among amino acid sequence, conformation and activity-selectivity, a combinatorial library consisting of *N*-methylated-SHU9119 analogues with single and multiple *N*-methylation in the core cyclic sequence of SHU9119 has been designed, synthesized and characterized *in vitro.* Syntheses were performed in analogy to the synthesis of the library of *N*-methylated MT-II peptides.⁶⁰

### SUMMARY OF THE INVENTION

Melanocortin receptors (hMC1R, hMC3R, hMC4R and hMC5R), due to their structural similarity have been challenging targets to develop ligands that are explicitly selective to each of their subtypes, which is further complicated by the presence of separate biological agonist and antagonist responses for the receptor system demanding for function specific ligands too. To modulate the conformational preferences of the melanocortin ligands and improve the biofunctional agonist-antagonistic activities and selectivities, the present invention applies a backbone *N-*methylation approach on SHU9119, a non-selective cyclic peptide antagonist at hMC3R and hMC4R. Systematic *N*-methylated derivatives of SHU9119, with all possible backbone *N*-methylation combinations have been synthesized and examined for their binding and functional activities towards melanocortin receptor subtypes 1, 3, 4 and 5 (hMCRs).

Several *N*-methylated analogues which have selective and potent agonists or antagonists activity for the hMC1R or hMC5R; or selective antagonist activity for the hMC3R are discovered from the library. The selective hMC1R ligands show strong binding for human melanoma cells. The present invention features the first universal antagonist for all subtypes of hMCRs, which will be of critical importance to modulate the melanocortin system with endogenous agonists. Structure Activity Relation (SAR) studies of selective ligands and chimeric human melanocortin 3 receptor lead to new insights for hMC3R selectivity. No correlation could be seen between the SHU9119 and the previous MT-II libraries, with respect to the *N*-methylation pattern and switching of agonistic or antagonistic activities.

Conformational studies in solution of the selected ligands have revealed that the peptides have an anti-parallel β-sheet structure and the pharmacophore (His⁶-nal⁷-Arg⁸-Trp⁹) occupies a βII'-turn like region with the turn centered about nal⁷-Arg⁸. The analogues with different selectivities however showed distinct differences in the spatial arrangement of individual amino acid side chains. The docking studies do not show any interactions between the *N*-methyl groups and the receptors, implying that the role of *N*-methyl groups is primarily to reorganize the conformation of the peptide backbone. The present invention signifies the sensitivity of the melanocortin system to subtle changes in preferred backbone conformation and orientation of the functional groups in ligands to define the agonistic and antagonistic activity for a given peptide sequence.

### BRIEF DESCRIPTION OF THE DRAWINGS

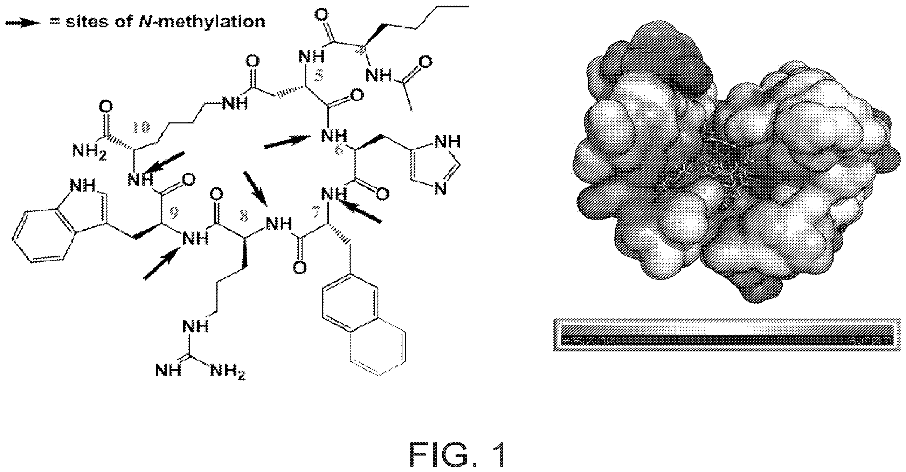

FIG. 1 shows a structure of SHU9119 peptide. The arrows indicate the sites of *N*-methylation in analogues synthesized with all combinations examined systematically.

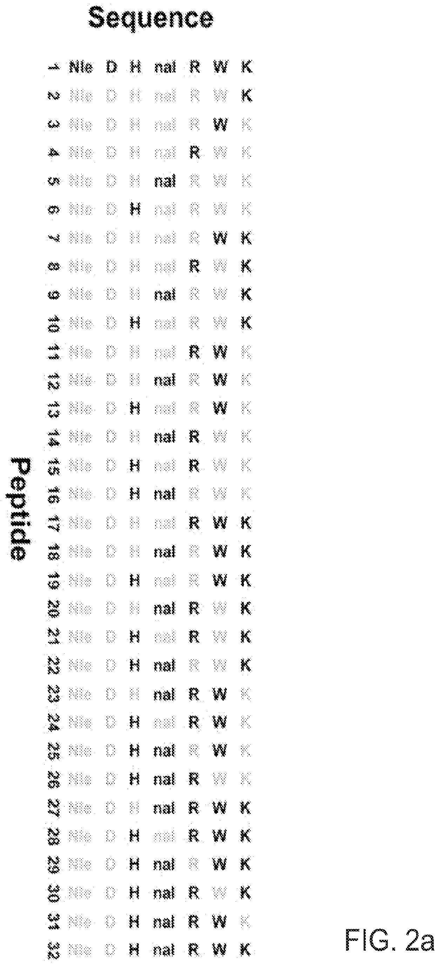

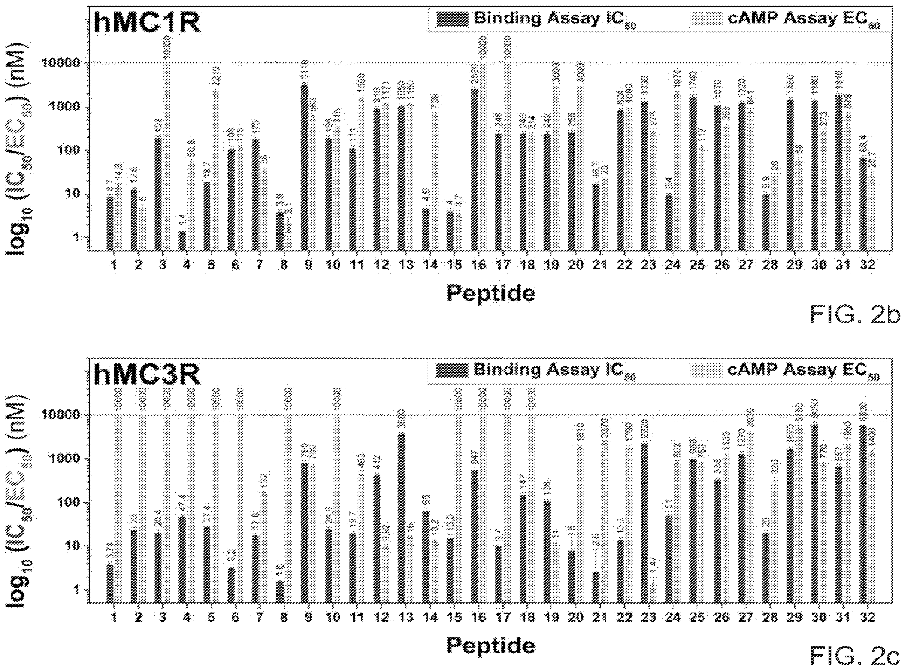

FIG. 2a shows the amino acid sequences for SHU9119 (1) and *N*-methylated SHU9119 analogues (peptides 2-32; with the site of *N*-methylation highlighted in bold font. FIGs. 2b to 2e respectively are histograms showing the results for IC50 from binding assay (black bars) and EC50 from cAMP assay (light grey bars) at hMC1R, hMC3R, hMC4R and hMC5R. For a convenient comparison, the IC₅₀ and EC₅₀ are plotted in log₁₀ scale with error bars also represented. However, the absolute IC₅₀ and EC₅₀ values are indicated above each bar. For more numeric details including the binding efficiency and ligand induced functional activity compared to MTII (Act%) are shown in Table 4 and Table 5. IC₅₀= concentration of peptide at 50% specific binding (*N*=4) and EC₅₀ = Effective concentration of peptide that was able to generate 50% maximal intracellular cAMP accumulation (*N*=4).

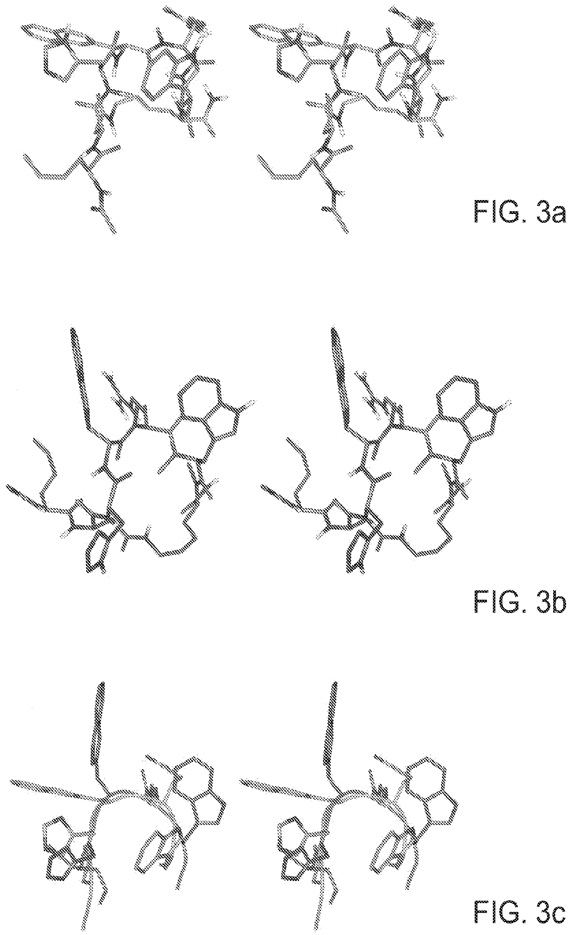

FIGs. 3a to 3c show stereo views of the NMR structure of peptides 15 (3a, pink); 17 (3b, green) and the overlay of the β-turn like His⁶-nal⁷-Arg⁸-Trp⁹ region from peptides 15 and 17 (3c). The ribbons along the backbones highlight the secondary structural features. For a clearer view, the non-polar hydrogens in FIG. 3a and 3b, whereas all the hydrogens and main chain in FIG. 3c are not shown.

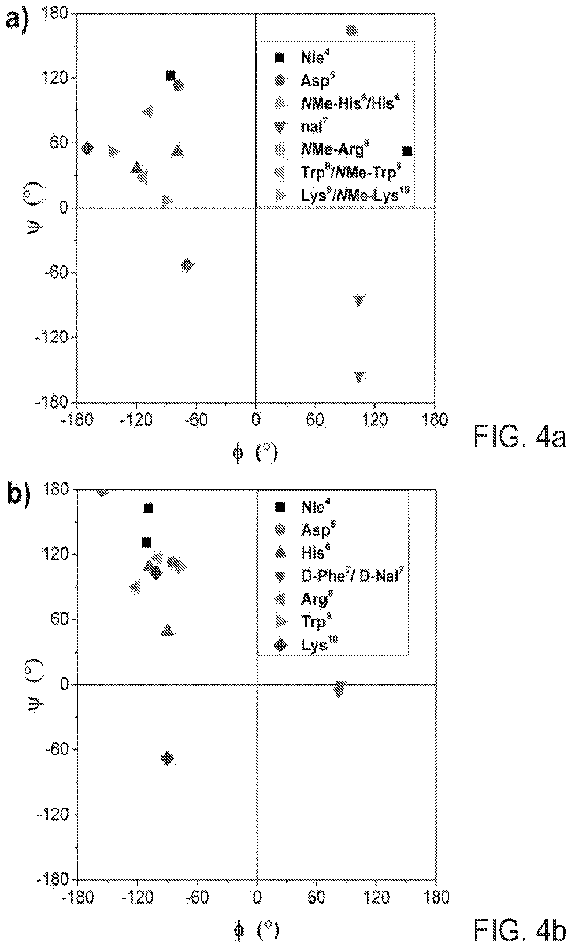

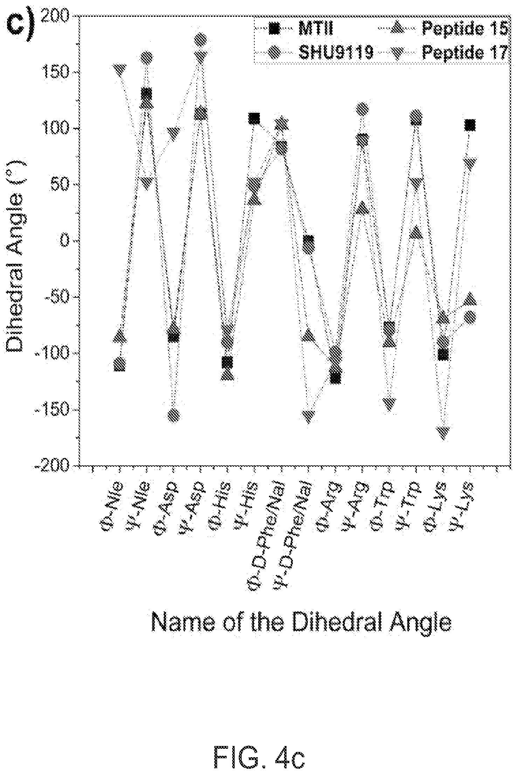

FIGs. 4a to 4c shows Ramachandran plots that represent the (Φ,Ψ) dihedral space of constituent amino acids and corresponding secondary structural information, generated for (4a) peptides 15 and 17 based on their NMR structures; (4b) for peptides MT-II and SHU9119. FIG. 4c is a graph showing the site specific variations in the dihedral space of the above four peptides.

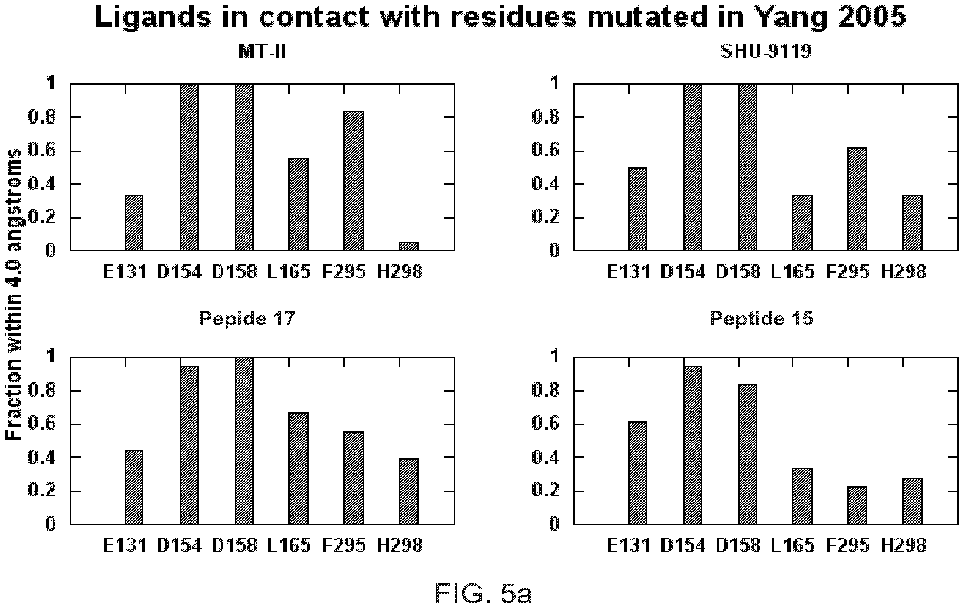

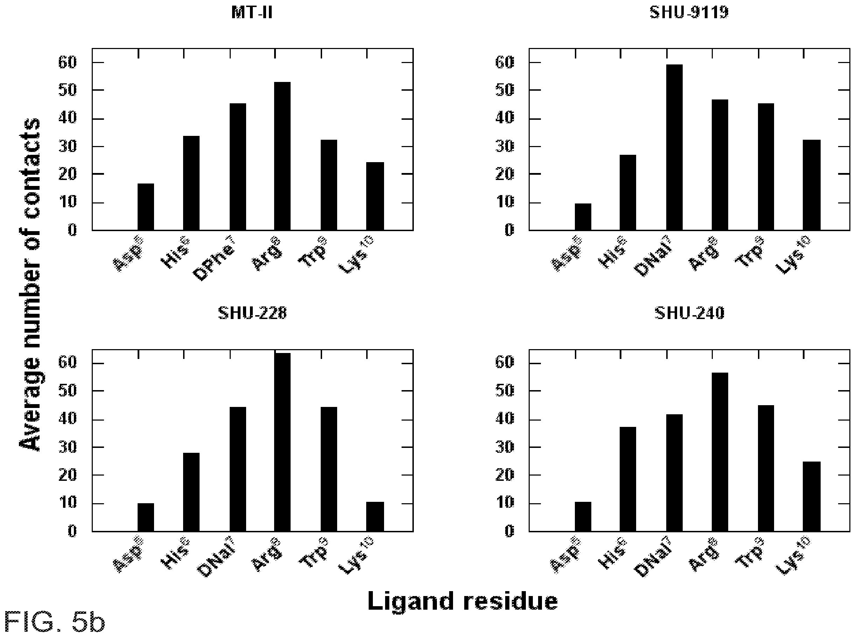

FIG. 5a shows ligands in contact with residues from the chimeric hMC3R. 1) F295 is contacted by MT-II in >80% of results, with less than 60% in SHU9119 /Peptide 17 and about 20% in Peptide 15, indicate that F295 is important for the agonist activity of hMC3R. 2) D154/D158 are contacted >83%, but almost 100% by each ligand, indicate that D154/D158 are important for the binding to the hMC3R; 3) H298 abolishes binding affinity in bioassay, but highest contact rate is 40%, usually closer to 20%. 4) E131 may play more important role in SHU9119 binding with 50-60 contact rates, whereas in MT-II has the lowest rate (35%). D-Phe-Arg-Trp is essential to binding with highest contact % rates. FIG. 5b shows docking results on interaction of peptide ligands with hMC3R showing the amino acid wise cumulative contacts with residues of the hMC3R. Arg⁸ has the highest contacts at hMC3R with nearly all four peptides except SHU9119. D-Nal(2')⁷ show more contacts at hMC3R compared to the rest of the three peptides since it is agonist of hMC1R and hMC5R; antagonist at hMC3R and hMC4R. Trp⁹ also has very high contacts (>40) at hMC3R (SHU9119, peptide 15 and 17) except for universal agonist MT-II, indicating that Trp⁹ plays an important role of the hMC3R antagonist activity.

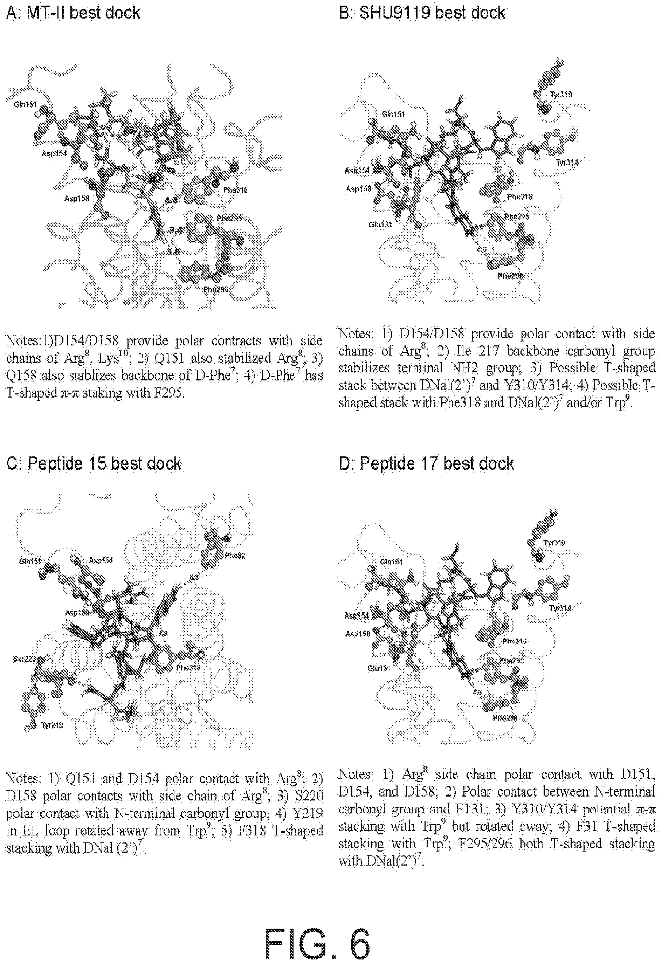

FIG. 6 shows docking results showing the peptide ligands binding with hMC3R. Six potential binding sites of hMC3R (E131, D154, D158, L165, F295, H298) are chosen to interact with the peptide ligands. Cluster represents unique docked conformation for two sets of docking runs for each ligand. Two runs multiplied by nine clusters equals18 total conformations.

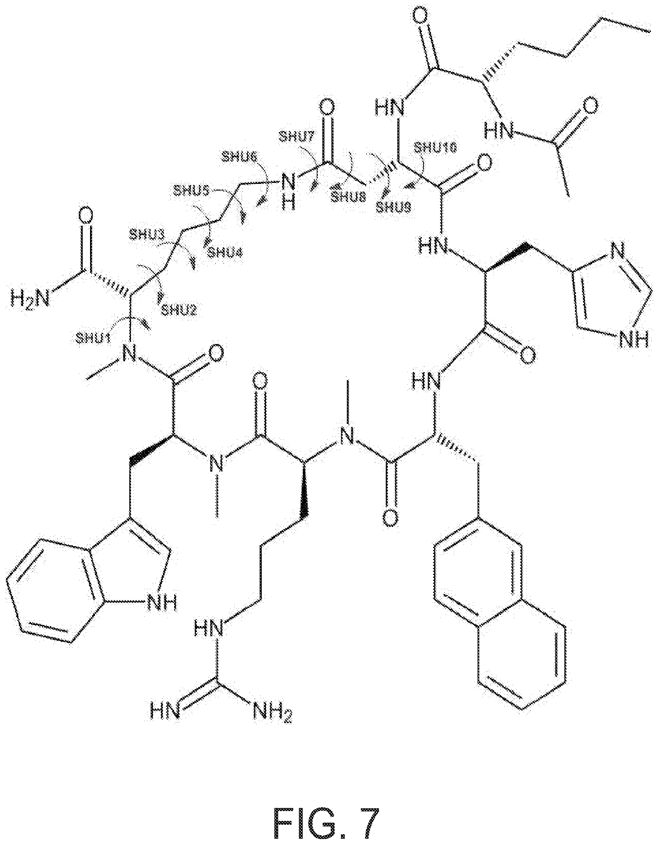

FIG. 7 shows names of the dihedral angles for the Lys10-Asp5 side chain cyclized region (as shown for peptide 17) of SHU9119.

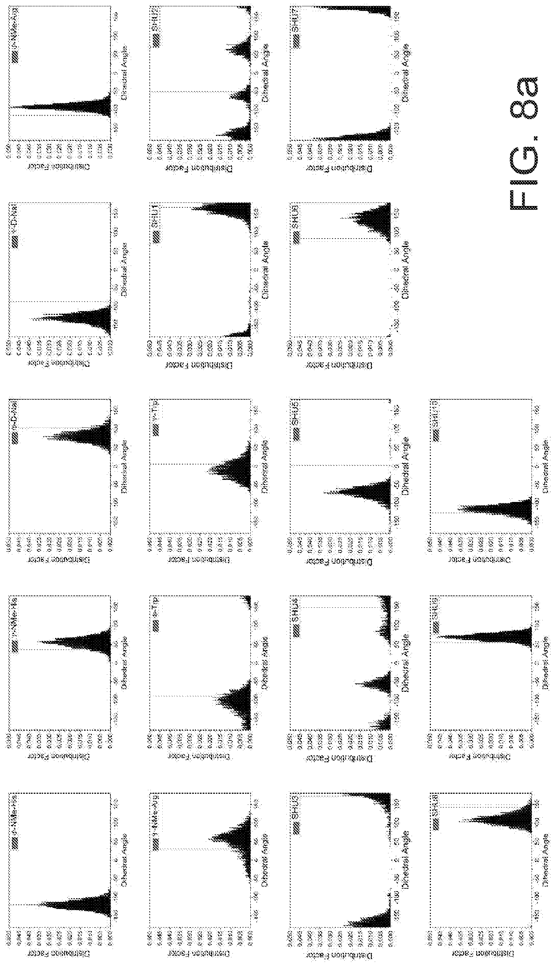

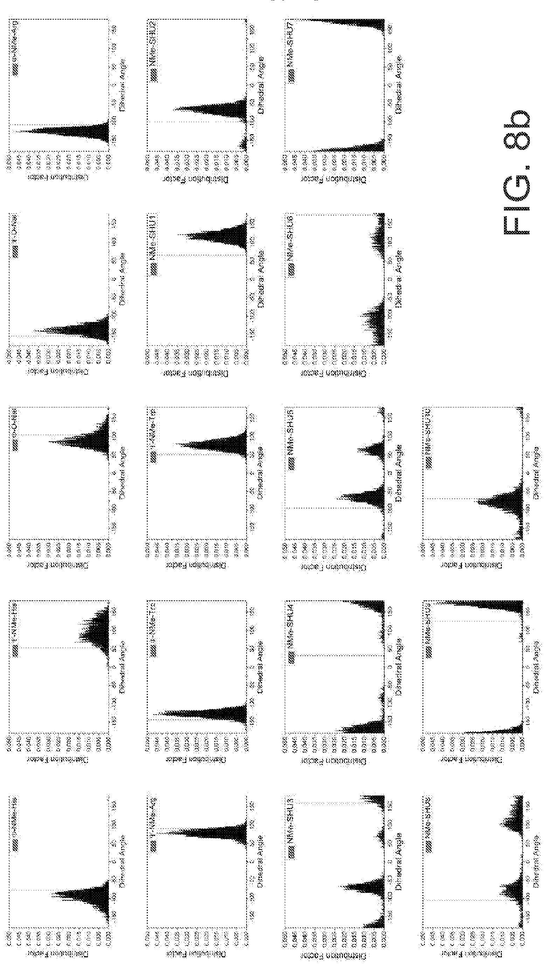

FIG. 8a shows histograms showing the backbone dihedral angle distribution in fMD (with restraints) trajectory of 15. The vertical red lines in each graph indicate the corresponding dihedral angle values as measured from the input DG structure. FIG. 8b shows histograms showing the backbone dihedral angle distribution in fMD (without restraints) trajectory of 17. The vertical red lines in each graph indicate the corresponding dihedral angle values as measured from the input DG structure.

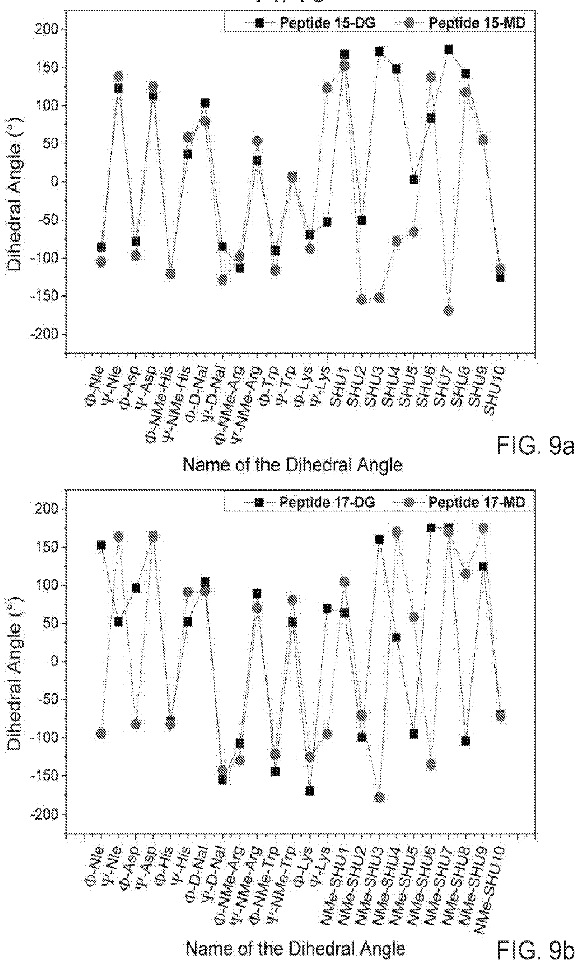

FIG. 9a shows overlay of the dihedral angles measured for 15 from its DG structure and the mean structure of the most dominant cluster represented in corresponding MD calculations. FIG. 9b shows overlay of the dihedral angles measured for 17 from its DG structure and the mean structure of the most dominant cluster represented in corresponding MD calculations.

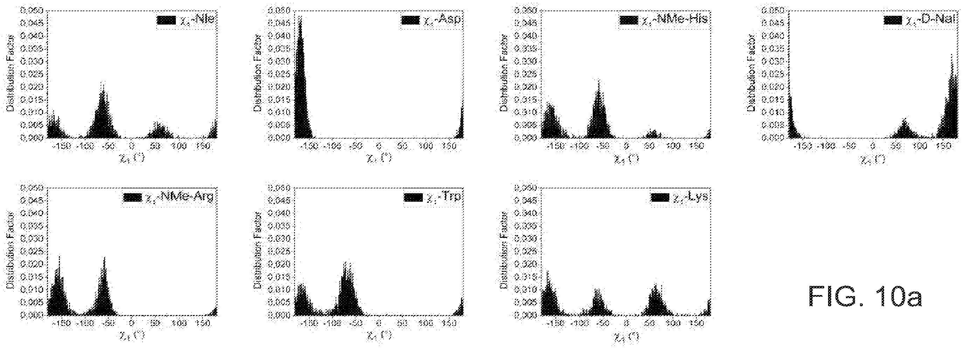

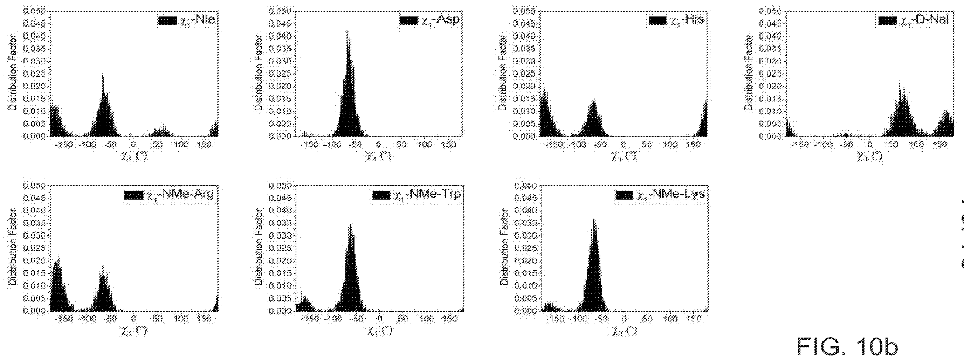

FIG. 10a shows χ1 dihedral angles distribution for side chains of peptide 15 as calculated from the corresponding MD trajectory. FIG. 10b shows χ1 dihedral angles distribution for side chains of peptide 17 as calculated from the corresponding MD trajectory.

### DESCRIPTION OF PREFERRED EMBODIMENTS

Referring now to FIGs. 1- 10, the present invention features a backbone *N-*methylation approach on SHU9119, a non-selective cyclic peptide antagonist at hMC3R and hMC4R. Specifically, the present invention features the modified mealnocortin receptor modulators for either a medcial use as indicated in claim 1 or anon-medical and non-surgical use as indicated in claim 3. Systematic *N*-methylated derivatives of SHU9119, with all possible backbone *N*-methylation combinations have been synthesized and examined for their binding and functional activities towards melanocortin receptor subtypes 1, 3, 4 and 5 (hMCRs). Each methylated form grants a different effect to certain melanocortin receptors, either agonizing them or antagonizing them. This would potentially reduce the amount and severity of side effects that treatments involving the invention would incur.

As defined herein, the term "agonist" refers to compound that enhances a response. The agonist binds to the same site as the endogenous compound and produces the same type of signal, usually of equal or greater magnitude than the endogenous agent.

As defined herein, the term "antagonist" refers to compound that diminishes a response. The antagonist binds to the same site as the endogenous compound and diminishes or blocks the signal generated by the endogenous agent.

As defined herein, the term "*N*-methylation" refers to a form of alkylation wherein a methyl group, CH₃, replaces the hydrogen atom of the NH moiety in an amino acid.

As defined herein, the terms "therapeutically effective amount" means the amount of a compound that, when administered to a mammal for treating a condition, is sufficient to effect such treatment for the condition. The "therapeutically effective amount" will vary depending on the compound, the condition and its severity and body factors such as age, weight, etc., of the mammal to be treated.

As defined herein, the terms "Treating" or "treatment" of a condition includes: (1) preventing the condition, i.e., causing the clinical symptoms of the condition not to develop in a mammal that may be exposed to or predisposed to the condition but does not yet experience or display symptoms of the condition; (2) inhibiting the condition, i.e., arresting or reducing the development of the condition or its clinical symptoms; or (3) relieving the condition, i.e., causing regression of the condition or its clinical symptoms.

As used herein, the terms "those defined above" and "those defined herein" when referring to a variable incorporates by reference the broad definition of the variable as well as any narrow and/or preferred, more preferred and most preferred definitions, if any.

As used herein, the term "*NMe*" preceding any three-letter abbreviation for an amino acid, i.e. (*NMe*)Lys or (*NMe*)Trp, denotes the *N*-methylated form of the amino acid. As used herein, the term "Nle" refers to a Norleucine.

Methods described herein may be equivalently represented in a Swiss-type format. As a non-limiting example, "a method for treating a disease Y using compound X" may have a Swiss-type equivalent of "the use of compound X in curing disease Y". It is to be understood that other Swiss-type formats may be acceptable.

The specification of the US patent application No. 14/300,991 was filed on June10, 2014. Paragraphs [00100] to [00186] in pages 25-49 of the specification are incorporated herein in their entirety by reference. Moreover, any of the peptide-ligands from SEQ. ID NO. 4, SEQ. ID NO. 5, SEQ. ID NO. 9, SEQ. ID NO. 11, SEQ. ID NO. 18, and SEQ. ID NO. 19 of U.S. Patent Application No. 14/300,991 are incorporated herein by reference. For example, SEQ. ID NO. 18, and SEQ. ID NO. 19 of U.S. Patent Application No. 14/300,991 are incorporated herein by reference: H-Tyr-Val-Leu-Gly-His-DPhe-Arg-DNal(2')-Asp-Arg-Phe-Gly-NH₂ (SEQ ID NO:18) and H-Tyr-Val-ILe-Gly-His-DPhe-Arg-DNal(2')-Asp-Arg-Phe-Gly-NH₂ (SEQ ID NO:19).

### N-methylated derivatives of SHU9119: Peptide Sequence List

Peptide 1: Ac-Nle-c[Asp-His-nal-Arg-Trp-Lys]-NH₂ (SEQ. NO. 1)
Peptide 2: Ac-Nle-c[Asp-His-nal-Arg-Trp-(*NMe*)Lys]-NH₂ (SEQ. NO. 2)
Peptide 3: Ac-Nle-c[Asp-His-nal-Arg-(*NMe*)Trp-Lys]-NH₂ (SEQ. NO. 3)
Peptide 4: Ac-Nle-c[Asp-His-nal-(*NMe*)Arg-Trp-Lys]-NH₂ (SEQ. NO. 4)
Peptide 5: Ac-Nle-c[Asp-(*NMe*)His-(*NMe*)nal-Arg-Trp-Lys]-NH₂ (SEQ. NO. 5)
Peptide 6: Ac-Nle-c[Asp-(*NMe*)His-nal-Arg-Trp-Lys]-NH₂ (SEQ. NO. 6)
Peptide 7: Ac-Nle-c[Asp-His-nal-Arg-(*NMe*)Trp-(*NMe*)Lys]-NH₂ (SEQ. NO. 7)
Peptide 8: Ac-Nle-c[Asp-His-nal-(*NMe*)Arg-Trp-(*NMe*)Lys]-NH₂ (SEQ. NO. 8)
Peptide 9: Ac-Nle-c[Asp-His-(*NMe*)nal-Arg-Trp-(*NMe*)Lys]-NH₂ (SEQ. NO. 9)
Peptide 10: Ac-Nle-c[Asp-(*NMe*)His-nal-Arg-Trp-(*NMe*)Lys]-NH₂ (SEQ. NO. 10)
Peptide 11: Ac-Nle-c[Asp-His-nal-(*NMe*)Arg-(*NMe*)Trp-Lys]-NH₂ (SEQ. NO. 11)
Peptide 12: Ac-Nle-c[Asp-His-(*NMe*)nal-Arg-(*NMe*)Trp-Lys]-NH₂ (SEQ. NO. 12)
Peptide 13: Ac-Nle-c[Asp-(*NMe*)His-nal-Arg-(*NMe*)Trp-Lys]-NH₂ (SEQ. NO. 13)
Peptide 14: Ac-Nle-c[Asp-His-(*NMe*)nal-(*NMe*)Arg-Trp-Lys]-NH₂ (SEQ. NO. 14)
Peptide 15: Ac-Nle-c[Asp-(*NMe*)His-nal-(*NMe*)Arg-Trp-Lys]-NH₂ (SEQ. NO. 15)
Peptide 16: Ac-Nle-c[Asp-(*NMe*)His-(*NMe*)nal-Arg-Trp-Lys]-NH₂ (SEQ. NO. 16)
Peptide 17: Ac-Nle-c[Asp-His-nal-(*NMe*)Arg-(*NMe*)Trp-(*NMe*)Lys]-NH₂ (SEQ. NO. 17)
Peptide 18: Ac-Nle-c[Asp-His-(*NMe*)nal-Arg-(*NMe*)Trp-(*NMe*)Lys]-NH₂ (SEQ. NO. 18)
Peptide 19: Ac-Nle-c[Asp-(*NMe*)His-nal-Arg-(*NMe*)Trp-(*NMe*)Lys]-NH₂ (SEQ. NO. 19)
Peptide 20: Ac-Nle-c[Asp-His-(*NMe*)nal-(*NMe*)Arg-Trp-(*NMe*)Lys]-NH₂ (SEQ. NO. 20)
Peptide 21: Ac-Nle-c[Asp-(*NMe*)His-nal-(*NMe*)Arg-Trp-(*NMe*)Lys]-NH₂ (SEQ. NO. 21)
Peptide 22: Ac-Nle-c[Asp-(*NMe*)His-(*NMe*)nal-Arg-Trp-(*NMe*)Lys]-NH₂ (SEQ. NO. 22)
Peptide 23: Ac-Nle-c[Asp-His-(*NMe*)nal-(*NMe*)Arg-(*NMe*)Trp-Lys]-NH₂ (SEQ. NO. 23)
Peptide 24: Ac-Nle-c[Asp-(*NMe*)His-nal-(*NMe*)Arg-(*NMe*)Trp-Lys]-NH₂ (SEQ. NO. 24)
Peptide 25: Ac-Nle-c[Asp-(*NMe*)His-(*NMe*)nal-Arg-(*NMe*)Trp-Lys]-NH₂ (SEQ. NO. 25)
Peptide 26: Ac-Nle-c[Asp-(*NMe*)His-(*NMe*)nal-(*NMe*)Arg-Trp-Lys]-NH₂ (SEQ. NO. 26)
Peptide 27: Ac-Nle-c[Asp-His-(*NMe*)nal-(*NMe*)Arg-(*NMe*)Trp-(*NMe*)Lys]-NH₂; (SEQ. NO. 27)
Peptide 28: Ac-Nle-c[Asp-(*NMe*)His-nal-(*NMe*)Arg-(*NMe*)Trp-(*NMe*)Lys]-NH₂; (SEQ. NO. 28)
Peptide 29: Ac-Nle-c[Asp-(*NMe*)His-(*NMe*)nal-Arg-(*NMe*)Trp-(*NMe*)Lys]-NH₂; (SEQ. NO. 29)
Peptide 30: Ac-Nle-c[Asp-(*NMe*)His-(*NMe*)nal-(*NMe*)Arg-Trp-(*NMe*)Lys]-NH₂; (SEQ. NO. 30)
Peptide 31: Ac-Nle-c[Asp-(*NMe*)His-(*NMe*)nal-(*NMe*)Arg-(*NMe*)Trp-Lys]-NH₂; (SEQ. NO. 31)
Peptide 31: Ac-Nle-c[Asp-(*NMe*)His-(*NMe*)nal-(*NMe*)Arg-(*NMe*)Trp-(*NMe*)Lys]-NH₂; (SEQ. NO. 32)

Embodiment 1 (Reference): Disclosed is a modified melanocortin receptor modulator, the modulator comprising Ac-Nle-c[Asp-His-nal-Arg-Trp-Lys]-NH₂ ("SHU9119"), wherein at least one amino acid within the c[Asp-His-nal-Arg-Trp-Lys] portion of SHU9119 is *N*-methylated.
Embodiment 2 (Reference): Disclosed is further a method of modulating a melanocortin receptor, said method comprising administering to a mammal in need thereof at least one of the modified melanocortin receptor modulators according to Embodiment 1 in a therapeutically effective amount sufficient for stimulating melanin synthesis in said mammal. In some embodiments, administration of the modified melanocortin receptor modulators is by oral, nasal, or topical means, or by injection to the mammal. In some embodiments, the melanocortin receptor is an hMC1 receptor, an hMC3 receptor, an hMC4 receptor, an hMC5 receptor, or a combination thereof.
Embodiment 3 (Reference): Disclosed is further a method of modulating a melanocortin receptor for regulating a weight of a mammal, said method comprising administering to the mammal in need thereof at least one of the modified melanocortin receptor modulators according to Embodiment 1 in a therapeutically effective amount sufficient to bring about a determined satiation. In some embodiments, administration of the modified melanocortin receptor modulators is by oral, nasal, or topical means, or by injection to the mammal. In some embodiments, the melanocortin receptor is an hMC3 receptor, an hMC4 receptor, or a combination thereof.
Embodiment 4 (Reference): Disclosed is further a method of modulating a hMC4 receptor for stimulating sexual functions of a mammal, said method comprising administering to the mammal in need thereof at least one of the modified melanocortin receptor modulators according to Embodiment 1 in a therapeutically effective amount sufficient to bring about an enhanced and sustained sexual arousal. In some embodiments, administration of the modified melanocortin receptor modulators is by oral, nasal, or topical means, or by injection to the mammal.
Embodiment 5 (Reference): Disclosed is further a method of modulating a melanocortin receptor for regulating pain sensed by a mammal, said method comprising administering to the mammal in need thereof at least one of the modified melanocortin receptor modulators according to Embodiment 1 in a therapeutically effective amount sufficient to bring about sufficient pain relief. In some embodiments, administration of the modified melanocortin receptor modulators is by oral, nasal, or topical means, or by injection to the mammal. In some embodiments, the melanocortin receptor is an hMC1 receptor, an hMC5 receptor, or a combination thereof.
Embodiment 6: The present invention features a modified melanocortin receptor modulator for medical use of modulating a melanocortin receptor for regulating skin pigmentation of a mammal. The at least one of the modified melanocortin receptor modulators as indicated in claim 1 is provided in a therapeutically effective amount sufficient to bring about a desired skin coloration. In some embodiments, administration of the modified melanocortin receptor modulators is by oral, nasal, or topical means, or by injection to the mammal. The melanocortin receptor is an hMC1 receptor.
Embodiment 7 (Reference): Disclosed is further a method of modulating the hMC1 receptor for controlling the immune system of a mammal, said method comprising administering to the mammal in need thereof at least one of the modified melanocortin receptor modulators according to Embodiment 1 in a therapeutically effective amount sufficient to bring about stimulation of an immune response. In some embodiments, administration of the modified melanocortin receptor modulators is by oral, nasal, or topical means, or by injection to the mammal.
Embodiment 8 (Reference): Disclosed is further a method of modulating a melanocortin receptor for treating atherosclerosis in a mammal, said method comprising administering to the mammal in need thereof at least one of the modified melanocortin receptor modulators according to Embodiment 1 in a therapeutically effective amount sufficient to reduce atherosclerotic plaque inflammation and to improve vascular endothelial function. In some embodiments, administration of the modified melanocortin receptor modulators is by oral, nasal, or topical means, or by injection to the mammal. In some embodiments, the melanocortin receptor is an hMC1 receptor, an hMC3 receptor, or a combination thereof.
Embodiment 9 (Reference): Disclosed is further a method of modulating a melanocortin receptor for treating skin cancer in a mammal, said method comprising administering to the mammal in need thereof at least one of the modified melanocortin receptor modulators according to Embodiment 1 in a therapeutically effective amount sufficient to prevent proliferation of cancer cells. In some embodiments, administration of the modified melanocortin receptor modulators is by oral, nasal, or topical means, or by injection to the mammal. In some embodiments, the skin cancer is caused by over-exposure to ultraviolet radiation. In some embodiments, the melanocortin receptor is an hMC1 receptor, an hMC5 receptor, or a combination thereof.
Embodiment 10 (Reference): Disclosed is further a method of modulating a melanocortin receptor for treating a skin disease in a mammal, said method comprising administering to the mammal in need thereof at least one of the modified melanocortin receptor modulators according to Embodiment 1 in a therapeutically effective amount sufficient to bring about a prevention of symptoms or a cessation of the skin disease. In some embodiments, administration of the modified melanocortin receptor modulators is by oral, nasal, or topical means, or by injection to the mammal. In some embodiments, the melanocortin receptor is an hMC1 receptor, an hMC5 receptor, or a combination thereof. In some embodiments, the skin disease is a skin pigmentation disorder.
Embodiment 11 (Reference): Disclosed is further the use of the modified melanocortin receptor modulator according to Embodiment 1 for modulating a melanocortin receptor for stimulating melanin synthesis. In some embodiments, the melanocortin receptor is an hMC1 receptor, an hMC3 receptor, an hMC4 receptor, an hMC5 receptor, or a combination thereof.
Embodiment 12 (Reference): Disclosed is further the use of the modified melanocortin receptor modulator according to Embodiment 1 for modulating a melanocortin receptor for regulating weight by bringing about a determined satiation. In some embodiments, the melanocortin receptor is an hMC3 receptor, an hMC4 receptor, or a combination thereof.
Embodiment 13 (Reference): Disclosed is further the use of the modified melanocortin receptor modulator according to Embodiment 1 for modulating an hMC4 melanocortin receptor for stimulating sexual functions by bringing about an enhanced and sustained sexual arousal.
Embodiment 14 (Reference): Disclosed is further the use of the modified melanocortin receptor modulator according to Embodiment 1 for modulating a melanocortin receptor for regulating pain by bringing about sufficient pain relief. In some embodiments, the melanocortin receptor is an hMC1 receptor, an hMC5 receptor, or a combination thereof.
Embodiment 15: The present invention also features the non-medical and non-surgical use of the modified melanocortin receptor modulator as indicated in claim 3 to bring about a desired skin coloration. The melanocortin receptor is an hMC1 receptor.
Embodiment 16 (Reference): Disclosed is further the use of the modified melanocortin receptor modulator according to Embodiment 1 for modulating an hMC1 receptor for controlling an immune system by bringing about a stimulation of an immune response.
Embodiment 17 (Reference): Disclosed is further the use of the modified melanocortin receptor modulator according to Embodiment 1 for modulating a melanocortin receptor for treating atherosclerosis by reducing atherosclerotic plaque inflammation and improving vascular endothelial function. In some embodiments, the melanocortin receptor is an hMC1 receptor, an hMC3 receptor, or a combination thereof.
Embodiment 18 (Reference): Disclosed is further the use of the modified melanocortin receptor modulator according to Embodiment 1 for modulating a melanocortin receptor for treating skin cancer by preventing proliferation of cancer cells. In some embodiments, the skin cancer is caused by over-exposure to ultraviolet radiation. In some embodiments, the melanocortin receptor is an hMC1 receptor, an hMC5 receptor, or a combination thereof.
Embodiment 19 (Reference): Disclosed is further the use of the modified melanocortin receptor modulator according to Embodiment 1 for modulating a melanocortin receptor for treating a skin disease by bringing about a prevention of symptoms or a cessation of the skin disease. In some embodiments, the melanocortin receptor is an hMC1 receptor, an hMC5 receptor, or a combination thereof. In some embodiments, the skin disease is a skin pigmentation disorder.

The modulator according to Embodiment 1 is a member selected from a group consisting of the following *N*-methylated derivatives of SHU9119:
(SEQ. No. 3) Ac-Nle-c[Asp-His-nal-Arg-(*NMe*)Trp-Lys]-NH₂;
(SEQ. No. 8) Ac-Nle-c[Asp-His-nal-(*NMe*)Arg-Trp-(*NMe*)Lys]-NH₂;
(SEQ. No. 9) Ac-Nle-c[Asp-His-(*NMe*)nal-Arg-Trp-(*NMe*)Lys]-NH₂;
(SEQ. No. 10) Ac-Nle-c[Asp-(*NMe*)His-nal-Arg-Trp-(*NMe*)Lys]-NH₂;
(SEQ. No. 13) Ac-Nle-c[Asp-(*NMe*)His-nal-Arg-(*NMe*)Trp-Lys]-NH₂;
(SEQ. No. 15) Ac-Nle-c[Asp-(*NMe*)His-nal-(*NMe*)Arg-Trp-Lys]-NH₂;
(SEQ. No. 16) Ac-Nle-c[Asp-(*NMe*)His-(*NMe*)nal-Arg-Trp-Lys]-NH₂;
(SEQ. No. 17) Ac-Nle-c[Asp-His-nal-(*NMe*)Arg-(*NMe*)Trp-(*NMe*)Lys]-NH₂;
(SEQ. No. 19) Ac-Nle-c[Asp-(*NMe*)His-nal-Arg-(*NMe*)Trp-(*NMe*)Lys]-NH₂;
(SEQ. No. 28) Ac-Nle-c[Asp-(*NMe*)His-nal-(*NMe*)Arg-(*NMe*)Trp-(*NMe*)Lys]-NH₂; or
(SEQ. No. 32) Ac-Nle-c[Asp-(*NMe*)His-(*NMe*)nal-(*NMe*)Arg-(*NMe*)Trp-(*NMe*)Lys]-NH₂.

In the invention, the modulator is a selective hMC1 receptor agonist is a member selected from a group consisting of the following *N*-methylated derivatives of SHU9119:
(SEQ. No. 8) Ac-Nle-c[Asp-His-nal-(*NMe*)Arg-Trp-(*NMe*)Lys]-NH₂;
(SEQ. No. 10) Ac-Nle-c[Asp-(*NMe*)His-nal-Arg-Trp-(*NMe*)Lys]-NH₂;
(SEQ. No. 15) Ac-Nle-c[Asp-(*NMe*)His-nal-(*NMe*)Arg-Trp-Lys]-NH₂; or
(SEQ. No. 28) Ac-Nle-c[Asp-(*NMe*)His-nal-(*NMe*)Arg-(*NMe*)Trp-(*NMe*)Lys]-NH₂.

A highly selective hMC1 receptor antagonist is:
(SEQ. No. 32) Ac-Nle-c[Asp-(*NMe*)His-(*NMe*)nal-(*NMe*)Arg-(*NMe*)Trp-(*NMe*)Lys]-NH₂.

A selective hMC3 receptor antagonist is a member selected from a group consisting of the following *N*-methylated derivatives of SHU9119:
(SEQ. No. 8) Ac-Nle-c[Asp-His-nal-(*NMe*)Arg-Trp-(*NMe*)Lys]-NH₂;
(SEQ. No. 15) Ac-Nle-c[Asp-(*NMe*)His-nal-(*NMe*)Arg-Trp-Lys]-NH₂; or
(SEQ. No. 17) Ac-Nle-c[Asp-His-nal-(*NMe*)Arg-(*NMe*)Trp-(*NMe*)Lys]-NH₂.

A highly selective hMC5 receptor agonist is:
(SEQ. No. 3) Ac-Nle-c[Asp-His-nal-Arg-(*NMe*)Trp-Lys]-NH₂.

A highly selective hMC5 receptor antagonist is a member selected from a group consisting of the following *N*-methylated derivatives of SHU9119:
(SEQ. No. 9) Ac-Nle-c[Asp-His-(*NMe*)nal-Arg-Trp-(*NMe*)Lys]-NH₂; or
(SEQ. No. 13) Ac-Nle-c[Asp-(*NMe*)His-nal-Arg-(*NMe*)Trp-Lys]-NH₂.

A selective universal antagonist for an hMC1 receptor, an hMC3 receptor, an hMC4 receptor, or an hMC5 receptor is a member selected from a group consisting of the following *N*-methylated derivatives of SHU9119:
(SEQ. No. 16) Ac-Nle-c[Asp-(*NMe*)His-(*NMe*)nal-Arg-Trp-Lys]-NH₂; or
(SEQ. No. 19) Ac-Nle-c[Asp-(*NMe*)His-nal-Arg-(*NMe*)Trp-(*NMe*)Lys]-NH₂.

### SAR studies of NMe-SHU9119 analogues

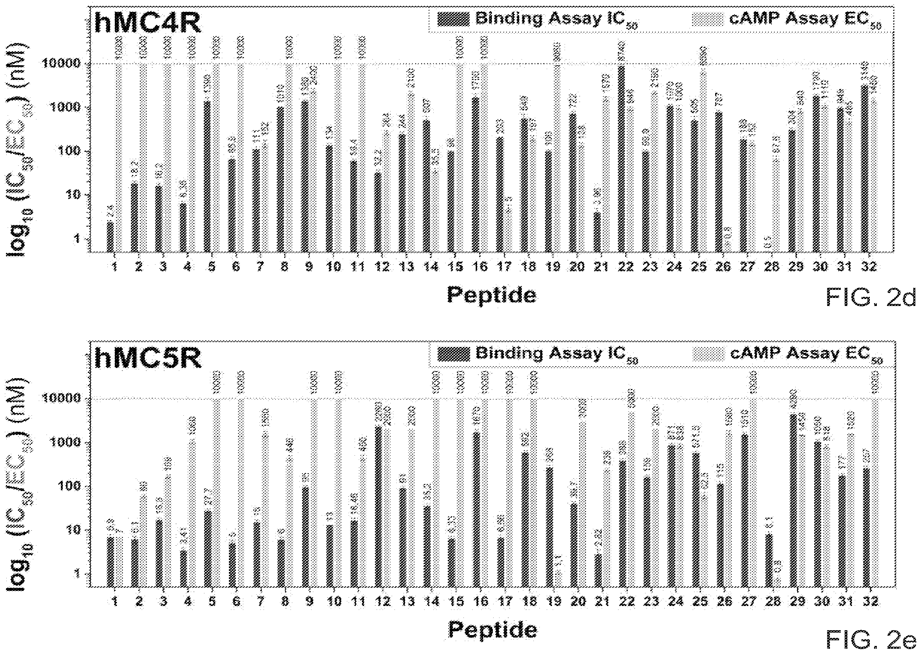

Effects of *N*-methylation on SHU9119 were evaluated on stably cloned hMCRs (hMC1R, hMC3R, hMC4R, and hMC5R) cell lines (HEK 293 cells) via competition binding assays and functional activity assays (cAMP assay). Competitive binding assays with [¹²⁵I]-[Nle⁴,D-Phe⁷]-α-MSH (NDP-α-MSH) on whole cells, and [³H]-adenylate cyclase on PKA were tested along with the application of standard MT-II molecules (Ac-Nle-c[Asp⁵, D-Phe⁷, Lys⁶]-α-MSH(4-10)NH₂ and SHU9119. The results are shown in Figure 2 (also refer to Tables 4 and 5). The pharmacological characterization for the binding efficiencies greater than 70% are defined as competitive binding, binding efficiency less than 70% are non-competitive binding. The pharmacological characterization for the potency of ligand used MTII as a standard full agonist is Act% equal to 100%. Ligands induced functional activity (cAMP level) compared to MTII generated cAMP are defined as Act%. Act% less than 30% are defined as No Activity; Act% between 30-50% is Partial Antagonist; Act% between 50-80% is Partial Agonist; and Act% greater than 80% is Agonist.

The first group of peptides are mono-*N*-methylated derivatives of SHU9119 (peptides 2-6) Figure 1 and 2a. In some embodiments, the competitive binding assays show that the whole profile of the mono-*N*-methylation at each position of the pharmacophore does not have a remarkable influence on the binding affinity towards hMCRs except for the hMC4R for *N*-methylation at the position 7 (*N*Me-nal⁷, peptide 5) implying that *N*Me-nal⁷ causes a drastical loss of binding affinity at the hMC4R. In some embodiments, cAMP assays show that peptide 3 with a *N*Me-Trp⁹ lead to a greater loss of functional activity (cAMP activity) at the hMC1R compared to the *N*Me-nal⁷ analogue 5, except at the hMC5R. Therefore, the single *N*-methylated derivatives of SHU9119 at the Trp⁹ position (Peptide 3) leads to a selective agonist for the hMC5R.

The second group of peptides are di-*N*-methylated derivatives in the core sequence of SHU9119 (peptides 7-16) Figure 1 and 2a. The first subgroup is *N*Me-Lys¹⁰+*N*MeXaa (Peptides 7-10, Xaa implies the rest of the amino acids in the cyclic structure of SHU9119 considered for *N*-methylation, Figure 1, Figure 2a). The results show that any site of *N*-methylation combined with *N*Me-Lys¹⁰ (Peptides 7-10, Figure 2) retains the same binding behavior as the first group for the hMC1R, hMC3R, hMC5R and loses the binding affinities 1-2 order of magnitude at the hMC4R; Peptide 9 loss the binding affinities towards all subtypes of melanocortin receptors due to the *N*Me-nal⁷. It is interesting to note that when both the positively charged amino acids (Arg⁸ and Lys¹⁰) are *N*-methylated at the peptide bond (peptide 8), the binding affinity for the hMC4R decreases by 3 orders of magnitude but increases at the hMC3R. Therefore, peptide 8 becomes a potent antagonist of the hMC3R and a potent agonist for hMC1R. This observation agrees with the previous studies using chimeric human melanocortin 4 receptor and truncation studies demonstrating that Arg⁸ is critical for the binding towards the hMC4R.^{57,58} The *N*-methylation of Arg⁸ changed the Φ, Ψ space and reduced the flexibility of the charged side chain group, which in turn reduced the binding affinity towards the receptor. In some embodiments, when *N*Me-nal⁷ is included (Peptide 9), binding affinity is drastically lost at the hMC1R, hMC3R, and hMC4R. Hence, Peptide 9, a very selective antagonist for the hMC5R is discovered.

The second subgroup of di-*N*-methylated SHU9119 analogues is *N*Me-Trp⁹+*N*MeXaa (peptides 7, 11-13). Peptide 7 retains the same binding affinities and functional activities as the mono-*N*-methylated derivatives of SHU9119. Peptide 11-13 all show antagonist activity based on the EC₅₀ and Act%. In the third subgroup consisting of *N*Me-Arg⁸+*N*MeXaa (peptides 8, 11, 14-15), the combination with *N*Me-Arg⁸ shows no significant changes in the binding behavior compared to the mono-*N-*methylated compounds, except for the hMC4R, as mentioned above. cAMP agonist activity at hMC5R is lost when it is combined with *N*Me-His. Interestingly, when *N*Me-Arg⁸ is combined with *N*Me-nal⁷, the hMC1R, hMC3R, hMC4R all show partial agonistic activities (peptide 14).

Among the fourth subgroup of *N*Me-nal⁷+*N*MeXaa peptides (peptides 9, 12, 14 and 16), the combination with *N*Me-nal⁷ showed a significant loss of the binding at all receptor subtypes. The fifth and last of the di-*N*-methylated SHU9119 compounds is *N*Me-His⁶+*N*MeXaa (peptides 10, 13, 15 and 16). When *N*Me-His⁶ is involved in di-*N*-methylated analogues, the agonist activity at the hMC5R is diminished compared to the first subgroup. When the di-*N*-methylated combination of *N*Me-His⁶ and *N*Me-Trp⁹ is used, the binding affinity is often reduced by one order of magnitude and the functional cAMP activities are lost at the hMC1R as was observed with the mono-*N*-methylated compounds (Peptides 7, 12 and 13). When two *N*-methylated aromatic residues (*N*Me-His⁶ and *N*Me-nal⁷, Peptide 16) are used as a combination, all of the agonist activities are lost and most of the binding affinities and cAMP activities are greatly diminished. However, the combination of *N*Me-His⁶ with the positive charged residues *N*Me-Lys¹⁰ and *N*Me-Arg⁶ increased selective agonist activities at the hMC1R (Peptides 10 and 15). Peptide 15 has a 6 fold selective potent antagonist activity at the hMC3R compared to that at hMC4R. Peptide 16 with all full binding efficiency towards all hMCRs is a universal antagonist for all subtype of melanocortin receptors. The fifth subgroup has also presented a moderate selective antagonist for the hMC5R (Peptide 13). Generally, when the aromatic amino acids are involved in dimethylation (Peptide 16), most of the binding affinities and cAMP activities are greatly diminished. Peptide 16 is a universal antagonist for the hMCRs.

The third group of peptides are the tri-*N*-methylated derivatives in the core sequence of SHU9119 (peptides 17-26) Figure 1 and 2a. Except for a few cases of dramatically increased selectivity, this group of peptides in general have shown significantly reduced binding affinities, which might be due to the hindrance caused by the multiple *N*-methylations that are involved; but in a few cases, there was dramatic increase in selectivity. Peptide 17 with the *N*-methylations at the positions of Arg⁸-Trp⁹-Lys¹⁰ lead to selective partial agonistic activity at the hMC4R and selective antagonist activities at the hMC3R (20 times more potent compare to the hMC4R). The combination of *N*Me-nal⁷, *N*Me-Trp⁹ and *N*Me-Lys¹⁰ (Peptide 18) show full agonist activity at the hMC1R and partial agonist at the hMC4R. The combination of *N*Me-His⁶, *N*Me-Trp⁹ and *N*Me-Lys¹⁰ (Peptide 19) show the good binding to all hMCRs but no activities to all hMCRs. Hence, Peptide 19 is another potent universal antagonist. The Peptide 20 also shows almost a universal antagonist activity as well (with very weak activity at the hMC3R).

The combination of *N*Me-nal⁷ with positive charged residue *N*Me-His⁶, *N*Me-Lys¹⁰ (Peptide 22); *N*Me-nal⁷, *N*Me-Arg⁸, *N*Me-Trp⁹ (Peptide 23), showed all functional activities are abolished, and the combination of *N*Me-His⁶, *N*Me-Trp⁹ and *N*Me-Arg⁸ (Peptide 24) showed selective partial agonist activity at the hMC1R and hMC5R. This is an observation in consistence with earlier MT-II *N*-methylation studies.⁶⁰ In this group, peptides 19-20 are antagonists or partial antagonist towards all hMCRs with reduced binding affinities compared to mono-*N*-methylated group of peptides (Peptide 2-6). Peptide 21 retains agonist activity at the hMC5R. It is noticed that, when *N*Me-nal⁷ is involved, nearly all of the binding affinities are lowered (Peptides 20, 22, 23, 25, 26).

The 4th group of peptides has five different tetra*-N-methylated* SHU9119 derivatives (peptides 27-31) Figure 1 and 2a. The studies show that the sites of N-methylation, when combined with *N*Me-nal⁷, reduce the binding affinity at all hMCRs. Only peptide 28 retains nanomolar binding at all hMCRs while cAMP activity show agonist activities at the hMC1R and hMC5R and antagonist activities at hMC3R and hMC4R, much like SHU9119. Peptide 28 exhibited a 0.5 nM binding to hMC4R, which is at least 16 folds stronger than its binding to other receptors and also the strongest binding exhibited by any other peptide in this library to any of the four discussed receptors.

The final penta*-N-methylated* compound 32 shows good binding at the hMC1R with an IC₅₀ of 68 nM and poor efficacy at the hMC5R (37%); and micromolar binding at the hMC3R and hMC4R. In the cAMP assay, the analogue was inactive at all hMCRs. Thus, a highly selective hMC1R antagonist is discovered.

### Comparison of N-methylation on MT-II and SHU9119

Earlier *N*-methylation studies on MT-II demonstrated that *N*-methylation of cyclized melanotropins increased the selectivities for hMCRs. For example, *N-*methylation of MT-II lead to the most selective agonist of hMC1R, which is also a specific biomarker for melanoma cells.⁶⁰ The present results showed that *N-*methylation of SHU9119 derivatives also leads to selective agonists and antagonists towards various hMCRs, but with a much broader spectrum of selectivity compared to *N*-methylated MT-II. The present invention features selective hMC1R agonists (peptide 8, 10, 15). Disclosed are further a most selective highly potent hMC1R agonist (peptide 32); selective antagonists for the hMC3R (peptide 8, 15, 17), and highly selective hMC5R agonists (peptide 3) and antagonists (peptide 9 and 13). Finally, disclosed are two unique universal antagonists (peptide 16 and 19) of all four hMCRs which is an important advancement for examining the biological roles of the endogenous agonist MSH system.

Direct comparison of binding and activity values, respectively, for the same methylation patterns shows that *N*-methylation influences structural changes of MT-II and SHU9119 in different ways. Influencing both compounds in a parallel manner leading to similar changes in binding and activity properties for each methylation pattern is probably not to be expected because the direct difference between MT-II and SHU9119 involves substitution of D-Phe⁷ by a bulky nal⁷. Since the bulky amino acid D-Nal(2') is presented in SHU9119 but not in MT-II, any *N*-methylation on these aromatic amino acids and combination with other *N*-methylated amino acids might have induced different conformational changes, which can dramatically change binding and functional selectivity properties. Introducing *N*-methylation on amide NHs of aromatic amino acids can be expected to re-orient the backbone conformation and side chain stacking (aromatic groups), all of which are important factors for receptor subtypes selectivity in the melanocortin receptors.

The following discussion is presented for purposes of illustration and description..

### Binding Assays of Selective hMC1R Ligands at Human Melanoma Cells

The above discussed SAR studies revealed that peptides 15 and peptide 28 are quite good selective agonists of the hMC1R. The present invention further investigates their binding and cAMP activities in human melanoma cells (A375). As shown in Table 1, these two peptides have also shown nanomolar binding affinities for the A375 melanoma cell.

| **Table 1.** Binding and cAMP activities of selected peptides at human melanoma cells (A375). | | | | | |
|---|---|---|---|---|---|
| **Peptide** | **Sequence** | **IC₅₀** | **Binding Efficiency** | **EC₅₀** | **Act %** |
| Peptide 15 | Ac-Nle-c[Asp-(*NMe*)His-nal-(*NMe*)Arg-Trp-Lys]-NH₂ | 14.8±2 | 100 | 22 ± 2.5 | NA |
| Peptide 28 | Ac-Nle-c[Asp-(*NMe*)His-nal-(*NMe*)Arg-(*NMe*)Trp-(*NMe*)Lys]-NH₂ | 8.7±1 | 100 | 20±2 | NA |
| MT-II | Ac-Nle-c[Asp-His-D-Phe-Arg-Trp-Lys]-NH₂ | 6.7 ± 2.9 | 100 ± 10 | 110 ± 0.6 | 100 |
| SHU9119 | Ac-Nle-c[Asp-His-nal-Arg-Trp-Lys]-NH₂ | : 100 ± 96 | 80 ± 34 | 3.3 ± 2.6 | NA |

### NMR Conformational Studies

To understand the conformational changes brought in by the *N*-methylation of SHU9119 and for a better interpretation of the different SAR (structure-activity relationship) between hMC3R and hMC4R, two representative peptides, 15 and 17 which both are antagonists at the hMC3R, selective agonist of hMC1R for peptide 15; selective partial agonist of hMC4R for peptide 17, are selected for structural characterization by NMR and MD. Thus obtained structural information has been further used for docking studies with hMC3R homology model.

NMR studies for the two peptides have been carried out in DMSO-*d*₆ solvent. The corresponding NMR spectroscopic structures in solution have been calculated by using distance geometry (DG)⁷⁴ program with NOE derived distances and ³*J*_{H}-coupling constant supported dihedral angles as structural restraints. In addition, the obtained structures are further analyzed by free or restrained (using the above derived NMR restraints) simulated annealing molecular dynamics (MD) calculations on GROMACS program⁷⁵⁻⁷⁷ by using the DG derived NMR structure as the starting conformation. The analysis resulted in a preferred backbone conformation (Figure **3**) for each of the peptides exempting the side chain cyclized region consisting of the Lys¹⁰-Asp⁴ residues.

A single set of ¹H chemical shift resonances (in DMSO-*d*₆ at 300K) with explicit ³*J*_{H} multiplicities and a good dispersion of backbone NH and Hα chemical shifts observed for these two peptides in the respective NMR spectra are indicative of a single predominant conformation and absence of slowly exchanging conformations on the time scales of NMR.⁷⁸ The final structures for both the peptides presented a *trans* configuration for all the peptide bonds (Figure **3**). Temperature coefficients (Δδ/ΔT); calculated for the backbone NHs over a temperature range of 295 K to 315 K, Table **8**) which are in the order < -3.00 ppb/K for all the NHs in both the peptides suggests their solvent accessibility and non-involvement in strong hydrogen bonding.

| Table 2. The (Φ,Ψ) dihedral angles at each of the constituent amino acids as measured from the NMR structures of the following peptides. | | | | | | | | |
|---|---|---|---|---|---|---|---|---|
| Compound | Nle4 | | Asp⁵ | | His⁶ | | D-Phe⁷/nal⁷ | |
| | | Ψ | Φ | Ψ | Φ | Ψ | Φ | Ψ |
| Peptide 15 | -85.7 | 122.3 | -78.1 | 113.1 | -119.5 | 36.4 | 103.4 | -84.9 |
| Peptide 17 | 152.9 | 52.1 | 96.3 | 164.4 | -78.7 | 51.9 | 104.1 | -155.0 |
| MTII | -111.0 | 131.0 | -85.0 | 113.0 | -108 | 109.0 | 84.0 | 0.0 |
| SHU9119 | -109.0 | 163.0 | -155.0 | 179.0 | -90.0 | 49.0 | 82.0 | -6.0 |
| MTII(*N*Me) | -101.0 | 109.1 | 71.4 | 143.7 | -97.8 | 78.4 | 96.1 | -125.9 |
| | | | | | | | | |

| Table 2. *continued* | | | | | | | | |
|---|---|---|---|---|---|---|---|---|
| Compound | Arg8 | | | Trp9 | | | Lys¹⁰ | |
| | Φ | | Ψ | Φ | Ψ | Φ | Ψ | |
| Peptide 15 | | -112.8 | 28.4 | -90.3 | | 6.5 | -69.2 | -52.8 |
| Peptide 17 | | -107.4 | 88.9 | -144.0 | | 51.7 | -169.5 | 69.3 |
| MTII | | -122.0 | 90.0 | -77.0 | | 108.0 | -101.0 | 103.0 |
| SHU9119 | | -99.0 | 117.0 | -79.0 | | 111.0 | -90.0 | -68.0 |
| MTII(*N*Me) | | -135.2 | 80.4 | -119.8 | | 62.6 | -113.7 | 0.4 |

For a comparative analysis of the conformations of 15, 17 and MT-II and SHU9119 related to α-MSH, the (Φ,Ψ) dihedral space of all the peptides are represented as Ramachandran plots⁷⁹ (Figure 4). Exempting the Φ-Nle⁴, Ψ-Nle⁴, and Φ-Asp⁵ which are external to the cyclic core of the peptides, the (Φ,Ψ) dihedral space for the cyclic region have mostly occupied the (-+ quadrant) of the Ramachandran plot corresponding to the β-sheet population. The (Φ,Ψ) angles for nal⁷ residues fall in (+- quadrant) diagonally opposite to the (-+ quadrant) which is meaningful considering its opposite stereo configuration. Although the (Φ,Ψ) distribution for basic MT-II and SHU9119 peptides is more centered in the β-sheet region, a downward shift of the overall distribution towards the overlapping β-sheet and α-helical regions is observed for 15 and 17. None of the sequential (Φ,Ψ) pattern matched perfectly with any of the standard β-turn types.^{80,81} However, the dihedral angles (Φ,Ψ) = (-78.7, 51.9) for His⁶ in peptide 17 are characteristic of an inverted γ-turn centered around it which facilitates for nal⁷-NH-Asp⁵-CO hydrogen bonding. In support of this, a temperature coefficient of -3.00 ppb/K for nal⁷-NH signifies a lower solvent accessibility and participation in a moderate hydrogen bonding. In contrary, the specific turn structure is disrupted in 15 as evident from the (Φ,Ψ) = (-119.5, 36.4) about His⁶. This might be due to the *N*-methylation of the His⁶-NH which brings in steric restriction factors and prevents an orientation of Asp⁵-His⁶ peptide bond that is appropriate for nal⁷-NH-Asp⁵-CO hydrogen bonding. In agreement is the temperature coefficient (-4.20 ppb/K) for nal⁷-NH in 15 proving its higher solvent accessibility in comparison to the corresponding nal⁷-NH in 17.

It can be stated that the amino acid stretch HiS⁶-nal⁷-Arg⁸-Trp⁹ is forming a β-turn like structure (rather than a loop which is often constituted by more amino acids) in both the peptides with a turnabout at nal⁷-Arg⁸. The turn residues are flanked by His⁶ and Trp⁹ amino acids on either side of the turn and are facing opposite to each other. The nucleation of turn around nal⁷-Arg⁸ can be explained by the conformational preference in general of consecutively located D-configured nal⁷ and *N*-methylated Arg⁸ amino acids. Corresponding dihedral space for the turn region in both the peptides can be noted from the Table 2 which are close to type II' β turn observed for D-Pro-L-Pro standard [(Φᵢ₊₁.Ψᵢ₊₁, Φᵢ₊₂,Ψᵢ₊₂) = (60°, -120°, -80°, 0)].^{82,83} A separation of Ca atoms of His⁶ and Trp⁹ by 6.12 Å and 7.17 Å, respectively in 15 and 17 is in agreement with general definition of turns and supports the current structure.^{80,81}

In both peptides, the backbone region opposite to the nal⁷-Arg⁸ is comprised of the side chains of Asp⁵-Lys¹⁰, which are linked via side chain cyclization. However, the distance between Ca atoms of Asp⁵ and Lys¹⁰ is measured to be 6.14 Å and 7.56 Å in 15 and 17, which is similar to the distance between Ca atoms of His⁶ and Trp⁹ in respective peptides. This suggests the propagation of nal⁷-Arg⁸ into an anti-parallel β-sheet structure along the His⁶ to Asp⁵ and Trp⁹ to Lys¹⁰ on either side. However, a shorter distance of ∼ 6 Å between the Ca atoms of oppositely located amino acids in 15 compared to ∼7 Å in 17 implies the constrained nature of the former structure. Apart from this, an increase in Ca separation from His⁶ and Trp⁹ to Asp⁵ and Lys¹⁰ in 17 implies the broadening of the structure along the length. This increase in girth may be is a consequence of the steric restrictions imposed by the three consecutive *N*-methyl groups in sequential amino acids namely Arg⁸, Trp⁹ and Lys¹⁰ of 17. The orientation of *N*Me in Arg⁸ is fixed by the conformational preorganization of turn forming nal⁷-Arg⁸ moiety whereas the arrangement of *N*Me of Trp⁹ into the cyclic core is necessitated both by the turn and the steric requirements from the *N*Me of Lys¹⁰. The absence of *N*Me on Trp⁹ for 15, therefore resulted in a narrower structure.

It is interesting to note the interplay of aromatic and Van der Waals interactions in the nal⁷-Arg⁸-Trp⁹ segment of 17. A long range π-π face to face aromatic interaction between the naphthyl group of nal⁷ and the indolyl group of Trp⁹ is directly evidenced by the strikingly very low chemical shift (1.989 ppm), for the *N-*Me on Arg⁸, which is directly sandwiched between the mentioned two aromatic groups. The observed chemical shift is so distinct that it is at least 0.7 ppm upfield of the chemical shift for any other *N*Me in both peptides. In contrast, a normal chemical shift value of 2.868 ppm for *N*Me of Arg⁸ in 15 implies the absence of such aromatic interactions and also different orientations for the corresponding side chains. The overall twist in the backbone conformation of the two peptides leading to a completely different arrangement of functional side chains can be noted from the secondary structure representation in Figure 3c.

The structures are further validated by MD studies. Though the MD calculations without any restraints have been sufficient to further validate the DG structure for 17, peptide 15 required usage of distance restraints within the cyclic region, which is speculated to be necessitated by the more constrained bent structure obtained for 15 by DG. Analysis of the MD trajectories for inter-proton distances (in Å, Tables 10 and 11) have shown that all the measured distances are in agreement with those calculated based on the NOE data, except for a very few distances connecting the protons in the *N*-methyl groups, the indole NH of Trp⁸ or the Nle⁴-NH. For all the complying cases, the mean value for each inter-proton distance has been measured to be within the ± 10% boundaries of the corresponding distance calculated from NMR and used in DG. The observed violations in the specific cases are attributed to the presence of Nle⁴-NH in a conformationally flexible region and the exchanging nature of the indole NH of Trp⁹. The stability of the backbone conformations for both peptides is further illustrated by the conserved dihedral angle distribution in the respective MD studies (Figures 8a to 9b), which are centered in the vicinity (maximum ±30°) of dihedral angles as measured from the corresponding DG structures. However, as expected, a flexibility in the distribution is observed for the side chain cyclized region.

The χ1 rotamer populations for the side chain conformations of all the amino acids are analyzed from the MD trajectories. They are also experimentally deduced based on the ³*J*_{Hα-Hβ}, heteronuclear couplings and NOE data of the stereospecifically assigned β-protons, wherever possible (see Supporting Information).⁸⁴ The absence of low ³*J*_{Hα-Hβ} coupling constants and their sum > 13 Hz in each of the amino acids suggests a complete predominance of χ₁=+60° conformation for L-amino acids and χ₁=-60° conformation for D-amino acids. The discrete ³*J*_{Hα-Hβ} values (but not with extreme difference of 9 Hz) observed for each residue therefore is a result of population mix mostly of χ₁=-60° (χ₁=+60° for D-amino acids) and χ₁=180° with a relative degree of preference within, which is represented also in the MD data. For Asp⁵, the NMR data imply χ₁=180° and χ₁=-60° as the most predominant conformations in 15 (*J*_{Hα-Hβ*pro*-R}=4.9 Hz, *J*_{Hα-Hβ*pro*-S}=10.2 Hz) and 17 (*J*_{Hα-Hβ*pro*-R}=9.1 Hz, *J*_{Hα-Hβ*pro*-S}=5.1 Hz), respectively, which is also reproduced in the MD data. As Asp⁵ side chain is cyclized with Lys¹⁰ side chain, and forms an integral part of the cyclic core, this distinctness for Asp⁵ χ₁ conformations in 15 and 17 may have been enforced on it by the conformational requirements of the cyclical core. NMR data for nal⁷ (with larger *J*_{Hα-Hβ*pro*-S}=9.0 Hz and 8.7 Hz coupling constants in 15 and 17, respectively) predicts χ₁=60° as the major conformation in both the peptides. Although the MD shows a similar population distribution in 15, a higher population of χ₁=180° is shown for 17. Lys¹⁰ in 15 has exhibited a dynamical nature with almost equally populated χ₁ rotamers in contrast to that in 17 with a strong preference for χ₁=-60°.

These varying peptide conformations for peptides 15 and 17, as derived from NMR stand as a proof for the conformational modulation in SHU9119 backbone that is generated by the *N*-methyl groups and are the key to the differential interaction with melanocortin receptor subtypes and corresponding activity responses. The implications of these structural dissimilarities that are responsible for distinct activities at receptors are further accounted by the docking studies.

The following is a non-limiting example of the present invention, and is presented for purposes of illustration and description.

### Docking Studies of Peptide 15 and Peptide 17

To further understand the difference of ligand-receptor interactions between the hMC3R and the hMC4R, docking simulations of MT-II, SHU9119, and peptides 15 and 17 were performed into the homology model of the hMC3R. Docking shows good overall agreement with mutation studies. A previous study from Chen et al. had identified several residues in MC3R that directly affect agonist/antagonist binding and receptor activity⁸⁵ (Figure 5, 6). Of these residues identified, all but two (D121 and D332) were proximal to the hMC3R binding pocket in the homology model. All clusters (total 18) for each ligand were analyzed to detect contacts within 4.0 Å of the mutated amino acids (Figure 6). Each ligand interacts with D154 and D158, consistent with the study by Chen et al., which showed that the mutation of D154 or D158 leads to dramatically decreased binding affinity and receptor signaling.⁸⁵ The docking results also show that D154/D158 have the highest contact within four peptides (MT-II, SHU9119, Peptide 15, Peptide 17) and indicate that D154/D158 are critically important for the ligand binding to the hMC3R. In the majority of cases, H298 was not in close contact with ligands, which is in contrary to the results by Chen et al. However, slight differences have also been seen between binding of MT-II and the SHU9119 N-methylated compounds: F295 was in contact with MT-II in over 80% of the clusters, whereas less than 60% of SHU9119 and peptide 17 clusters, and about 20% of peptide 15 clusters are in close proximity to F295, which indicates that F295 is important for agonist activity. These results agree with binding affinities of MT-II, SHU9119, peptide 17 and peptide 15 with 2nM, 3.7nM, 9.7nM and 15nM, respectively, at the hMC3R. The gradual decrease in contact rates with F295 at the hMC3R agrees with chimeric receptor studies from Yang's lab⁸⁵ as well and imply that F295 is critical for the agonist activity at the hMC3R. In addition, E131 did not play a significant role in MT-II binding (about 35% contact rate) but is observed to be in contact with more than half the clusters of the SHU ligands indicating that E131 is important for the antagonist activities of the SHU9119, peptides 15 and 17.

Another important aspect from the Chen et al. study is the identification of amino acid residues in the cyclic peptide ligands that were most responsible for binding and activity, showing that the -D-Phe⁷-Arg⁸-Trp⁹-tripeptide was the smallest fragment that could still bind and activate the MC3R.⁸⁵ Each of the current ligands confirm this result. D-Phe⁷ (or nal in the case of the SHU ligand), Arg⁸, and Trp⁹ are the residues with the highest average number of contacts (total number of contacts between ligand residue and any atom in MC3R, averaged over eighteen clusters) (Figure 5b). Slight differences exist among the ligands, as Arg⁸ has the most ligand-receptor contacts for MT-II, peptide 17, and peptide 15, whereas nal⁷ has the highest number of contacts for SHU9119.

Polar and aromatic interactions play important roles in ligand binding. Inspection of individual ligand-receptor complexes reveals subtle differences for MT-II, SHU9119, peptides 15 and 17. The best docked complex for the agonist MT-II (nM binding affinity (Kᵢ), forms polar contacts with D154 and D158 through the side chains of Arg⁸ (Figure 6A). In addition, polar contacts are formed between Q151 and Arg⁸ and between D158 and the backbone of D-Phe⁷. π-π stacking, which stabilizes interactions between aromatic amino acid side chains⁶⁷ are also present in the MT-II-MC3R complex. D-Phe⁷ inserts itself most deeply into the MC3R binding pocket, forming a T-shaped stacking with the aromatic side chains of F295, F296, and F318 on TM6 and TM7. In contrast, the antagonist, SHU9119 (nM Kᵢ) is rotated within the MC3R binding pocket. Polar interactions with D154 and D158 are still present, but are with Asp⁵ and Arg⁸, respectively (Figure 6B). An additional polar contact exists between the carbonyl group of the 1217 backbone and the terminal amino group of SHU9119. No penetration into the deepest part of the binding pocket is present with SHU9119, but a T-shaped stacking interaction is formed between nal⁷-F318-Trp⁹. Another possible T-shaped interaction could be formed between nal⁷ and either Y310 or Y314, which are located in the extracellular loop 3 (EL3), but its verification has been limited by the AutoDock Vina models which require a rigid protein backbone for the MC3R.

The methylated analogues of SHU9119, peptide 17 and 15, share some of the characteristic non-bonded interactions present for both MT-II and SHU9119 at the hMC3R. Peptide 17 (nM Kᵢ) forms an extensive network of polar contacts with Q151, D154, and D158 through its Arg⁸ side chain (Figure 6C). Additionally, there are also polar contacts existing on that side of the MC3R binding pocket between the backbone of Asp⁵ and the side chains of D154 and D158 as well as between the N-terminal carbonyl and the side chain of E131. Similar to MT-II, peptide 17 also wedges the nal⁷ residue into the deepest part of the binding pocket, forming a T-shaped stacking interactions with F295 and F296; however, Trp⁹ has stacking interactions with F318, similar to SHU9119. Again, Y310 and Y314 could potentially form stacking interactions with Trp⁹, but they lie just outside the 5 Å range typical of π-π stacking.

The poorest binder of the above four is peptide 15, most likely due to its orientation inside the binding pocket, different from that of MT-II, SHU9119, and peptide 17. Polar contacts were again formed with Arg⁸, but between the carbonyl backbone and side chains of Q151 and D154 (Figure 6D). Side chain polar contacts were only formed between Arg⁸ and D158. S220 also forms a polar contact with the acetyl group of the N-terminus. F318 is close enough to create stacking interactions, but is not orthogonal or parallel to nal⁷, a prerequisite for π-π stacking. Finally, Y219 could potentially form stacking interactions with Trp⁹, but is rotated away from the binding pocket.

Lacking selectivity and potency in agonistic or antagonistic properties of ligands for the melanocortin receptor subtypes is still the most difficult hurdle for application of these compounds in medicine. Mono- and multiple backbone *N-*methylations of SHU9119 peptides were investigated to achieve selective agonists and antagonists for melanocortin receptors via the conformational modulation in peptide backbone that is imparted by *N*-methyl steric constraints. This also is known to improve the pharmacokinetic profile of peptides in order to be used as drug leads. It turned out that the activity and selectivity profile induced by *N*-methylation is very different for the SHU9119 compared to a similar approach in MT-II. This is striking as both stem peptides differ only in the exchange of one amino acid (D-Nal⁷ instead of D-Phe⁷ in MT-II.

The systematic *N*-methylation of SHU9119 lead to the most selective antagonist of hMC1R (Peptide 32), which can be used as a powerful inhibitor for the treatment of melanoma. This strategy still retains several hMC1R agonists (peptide 8, 10 and 15) as previously described.⁶⁰ In the present invention, selective agonists for hMC5R (peptide 3) and also peptides 9 and 13 showed the most selective antagonistic activity found so far. The most selective antagonist for the hMC3R (peptide 17) known so far can potentially be used to treat obesity and diabetes. Apart from this, peptides 16, 19 are universal antagonist of the hMCR system, which is important to modulate the endogenous agonist MSH system. Molecular docking studies of MT-II, SHU9119, peptide 15 and peptide 17 reveal that Arg⁸ is critical for the binding towards D154, D158 at the hMC3R. The docking studies further support that the more the contact of Trp⁹ is towards the F295 at the hMC3R, the higher is the agonist activity that can be expected. These results strengthen the earlier discovered D-Trp⁸-γ-MSH as a selective agonist of hMC3R. Furthermore, E131 is seen to be important for the antagonist activities of the SHU9119 as well as of peptides 15 and 17. A complete structural characterization of all the important peptides discussed here for a more detailed correlation of melanocortin ligand conformation to the observed receptor discrimination is in progress, which information shall be crucial in structure based ligand designing for hMCRs.

Manifold of results obtained from multiply *N*-methylated SHU9119 peptide along with that from multiply *N*-methylated MT-II studies helped in understanding the finer details of the functional properties of melanocortin receptors and the bioactive conformational preferences of the ligands required by them. The studies further highlight the synthetic simplicity and impact of *N*-methylation strategy in fine tuning the conformational preferences of the ligands to achieve the desired biological effects. The studies, in general, anticipate a bright future for peptide chemistry toward the development as peptide therapeutic agents.

### Experimental Section

The following is a non-limiting example of the present invention, and is presented for purposes of illustration and description.

### Materials

*N*^{α}-Fmoc-amino acids, peptide coupling reagents, Rink amide MBHA resin and solvents were reagent grade and used without further purification unless otherwise specified. These chemicals were obtained from Aldrich, Novabiochem, Iris Biotech GmbH, Merck, NeoMPS, ORPEGEN Pharma and GLS. The following amino acids were used: Fmoc-Lys(Alloc)-OH, Fmoc-Trp(Boc)-OH, Fmoc-Arg(Pbf)-OH, Fmoc-nal-OH, Fmoc-His(Trt)-OH, Fmoc-Asp(OAllyl)-OH and Fmoc-Nle-OH. The polypropylene reaction vessels (syringes with frits) were purchased from B. Braun Melsungen AG. The purity of the peptides was checked by analytical reverse-phase HPLC using an Amersham Pharmacia Biotech Äkta Basic 10F with a ODS-A C18 (120 Å, 5 µm, 250 mm ×4.6 mm) column (Omnicrom YMC Europe GmbH) monitored at 220 and 254 nm and by high resolution mass spectral analysis.

### Synthesis

The SHU9119 analogues were synthesized manually by the Fmoc-SPPS and characterized using methods described in Doedens et al.⁶⁰

### NMR Conformational Studies

For NMR spectroscopic studies, approximately 1.5 to 2 mg of the compound was dissolved in 125 µl of DMSO-*d*6. The required NMR spectra were recorded at 300K on Bruker 500 MHz spectrometer equipped with TXI cryoprobe. 1H-1D, TOCSY, ROESY, HSQC, and HMBC NMR experiments have been acquired for each sample. 1H-selective homonuclear decoupling experiments were carried out to measure 3*J*H couplings. Furthermore, to estimate the solvent shielding or hydrogen bonding strengths of NH protons, the temperature dependency of NH chemical shifts was studied by acquiring 1H-1D spectra between 295 K and 315 K in steps of 5 K increments. Mixing times of 80 ms and 100 ms were used for TOCSY and ROESY experiments, respectively. HSQC spectra were recorded with a direct proton carbon coupling constant of 140 Hz, and HMBC spectra with a long-range 1H-13C coupling constant of 7 Hz. For HSQC spectra, a 13C composite pulse decoupling was utilized. 8k (except HSQC: 1k) data points were recorded in the direct dimension, 384 and 512 (heteronuclear spectra) in the indirect dimension. For all spectra a 1.5 s relaxation delay was used after every transient. Exponential / square sine window functions were used for spectra apodization.

**Proton-proton internuclear distances for structure calculation:** ROE cross peaks in corresponding ROESY spectra recorded in various solvents were integrated by using box method in SPARKY software. These integrated volumes of ROE cross peaks were converted to proton-proton internuclear distances by linear approximation method. Thus calculated distances were then relaxed by ±10 to generate upper and lower distance bounds to account for experimental and simulation uncertainties.

### Distance Geometry (DG) Calculations

Metric matrix DG calculations were carried out with a home-written distance geometry program utilizing random metrication. The above calculated experimental distance restraints which are more restrictive than the geometric distance bounds (holonomic restraints) were used to create the final distance matrix. 50 structures were calculated for each system. The structures were then verified and checked for violations if any with respect to the given experimental distance restraint inputs. The structures that best satisfied the distance inputs were then taken forward for MD simulations.

### Biological Activity Assays

**Receptor Binding Assay.** Competition binding experiments were carried out using both of cloned cell line and melanoma cells (A375, ATCC). The whole HEK293 cells stably expressing human MC1, MC3, MC4, and MC5 receptors as described before. HEK293 cells transfected with hMCRs were seeded on 96-well plates 48 hours before assay (50,000 cells/well). For the assay, the cell culture medium was aspirated and the cells were washed once with a freshly prepared MEM buffer containing 100% minimum essential medium with Earle's salt (MEM, GIBCO), and 25 mM sodium bicarbonate. Next, the cells were incubated for 40 min at 37°C with different concentrations of unlabeled peptide and labeled [¹²⁵I]-[Nle⁴,DPhe⁷]-α-MSH (Perkin-Elmer Life Science, 20,000 cpm/well, 33.06 pM) diluted in a 125 µL of freshly prepared binding buffer containing 100% MEM, 25 mM HEPES (pH 7.4), 0.2% bovine serum albumin, 1 mM 1,10-phenanthrolone, 0.5 mg/L leupeptin, 200 mg/L bacitracin. The assay medium was subsequently removed, the cells were washed once with basic medium, and then lysed by the addition of 100 µL of 0.1M NaOH and 100 µL of 1% Triton X-100. The lysed cells were transferred to 12×75 mm borosilicate glass tubes, and the radioactivity was measured by a Wallac 1470 WIZARD Gamma Counter. The results are shown in Table **2.**

**Adenylate Cyclase Assay.** HEK293 cells transfected with human melanocortin receptors were grown to confluence in MEM medium (GIBCO) containing 10% fetal bovine serum, 100 units/mL penicillin and streptomycin, and 1 mM sodium pyruvate. The cells were seeded on 96-well plates 48 hours before assay (50,000 cells/well). For the assay, the cell culture medium was removed and the cells were rinsed with 100 µL of MEM buffer (GIBCO). An aliquot (100 µL) of the Earle's balanced salt solution with 5 nM isobutylmethylxanthine (IBMX) was placed in each well for 1 min at 37°C. Next, aliquots (25 µL) of melanotropin peptides of varying concentration were added, and the cells were incubated for 3 min at 37°C. The reaction was stopped by aspirating the assay buffer and adding 60 µL ice-cold Tris/EDTA buffer to each well, then placing the plates in a boiling water bath for 7 min. The cell lysates were then centrifuged for 10 min at 2,300 x g. A 50 µL aliquot of the supernatant was transferred to another 96-well plate and placed with 50 µL [³H] cAMP and 100 µL protein kinase A (PKA) buffer in an ice bath for 2-3 hours. The PKA buffer consisted of Tris/EDTA buffer with 60 µg/mL PKA and 0.1% bovine serum albumin by weight. The incubation mixture was filtered through 1.0 µm glass fiber filters in MultiScreen™-FB 96-well plates (Millipore, Billerica, MA). The total [³H] cAMP was measured by a Wallac Micro β- TriLux 1450 LSC and Luminescence Counter (PerkinElmer Life Science, Boston, MA). The cAMP accumulation data for each peptide analogue was determined with the help of a cAMP standard curve generated by the same method as described above. IC₅₀ and EC₅₀ values represent the mean of two experiments performed in triplicate. IC₅₀ and EC₅₀ estimates and their associated standard errors were determined by fitting the data using a nonlinear least squares analysis, with the help of GraphPad Prism 4 (GraphPad Software, San Diego, CA). The maximal cAMP produced at 10 µM concentration of each ligand was compared to the amount of cAMP produced at 10 µM concentration of the standard agonist MT-II, and is expressed given as percentage(as % max effect) in the Table 3. The antagonist properties of the lead compounds were evaluated by their ability to competitively displace the MT-II agonist in a dose-dependent manner, at up to 10 µM.

**Data Analysis.** IC₅₀ and EC₅₀ values represent the mean of two experiments performed in triplicate. IC₅₀ and EC₅₀ estimates and their associated standard errors were determined by fitting the data using a nonlinear least squares analysis, with the help of GraphPad Prism 5 (GraphPad Software, San Diego, CA).

**Homology model** Chain R of the crystal structure of the activated β₂-adrenergic receptor (PDB ID 3SN6) was used as the template for secondary structural alignment in constructing the multiple sequence alignment (MSA). Seven other GPCR crystal structure sequences were included in the MSA (β₁-adrenergic receptors 2VT4 and 2Y00, A₂A receptors 2YDV, 3EML, and 3PWH, and β₂-adrenergic receptors 3KJ6 and 3P0G). In addition to the sequence from *Homo sapiens* (RefSeq ID NP_063941), MC3R sequences (AFH58736, AAI62747, AFH58735, NP_032587, ACK98821, AFH58734, NP_001020441, AAS66720, AFK25142, ACK98822) from ten other organisms were used. ClustalX⁸⁶ was used to generate the initial MSA, and manual alignment to the most highly conserved residue in each transmembrane helix (1.50: Asn93; 2.50: Asp121; 3.50: Arg179; 4.50: Trp206; 5.50: Met236; 6.50: Pro294; 7.50: Pro333, MC3R numbering) was carried out in SeaView.⁸⁷ 3SN6 was also chosen as the template structure for homology model construction. MODELLER⁸⁸ was used to generate a set of ten MC3R models. The model that retained alpha-helical structure for all transmembrane helices, as well as the characteristic outward helical tilt and rotation in TM5 and TM6 of activated GPCRs, was selected for further loop refinement. All extracellular loops were simultaneously optimized using MODELLER, and the model chosen for docking studies had all extracellular loops pulled away from the active site. This was chosen because previous studies had shown that extracellular loops had no effect on ligand binding to the MC3R.⁸⁹ Cytoplasmic loops were not optimized due to the fact that they are distal from the binding pocket.

***Ligand construction.*** The 2-D MT-II structure was obtained in .sdf format from PubChem⁴ and converted to three-dimensions and minimized in Avogadro.⁹⁰ SHU9119, peptide **15,** and peptide **17** structures were obtained based on NMR data from the Hruby and Kessler labs.

***Docking protocol.*** Receptors and ligands were prepared for docking runs using the AutoDockTools4 (ADT) graphical user interface.⁹¹ ADT was used to add hydrogen atoms, assign partial charges and atom types, and merge non-polar hydrogen atoms into their respective heavy-atom bonded partners for the MC3R homology model. Default settings were used to define rotatable bonds in each of the ligands to allow flexibility. The AutoDock/Vina plug-in for PyMOL⁹² was used to set parameters for the docking search space, which was a cube 33.75 Å in each direction, centered on the MC3R binding pocket.

AutoDock Vina⁹³ was used to perform molecular docking of ligands into the MC3R binding pocket. Default settings were used, unless otherwise specified. Each Vina run produced nine clusters, and each ligand was run twice, for a total of eighteen clusters per ligand. Results were visualized in PyMOL using the AutoDock/Vina plug-in and analyzed for protein-ligand contacts with python scripts. AutoDock4⁹³ was used to calculate the binding energy and inhibition constants of each cluster from Vina.

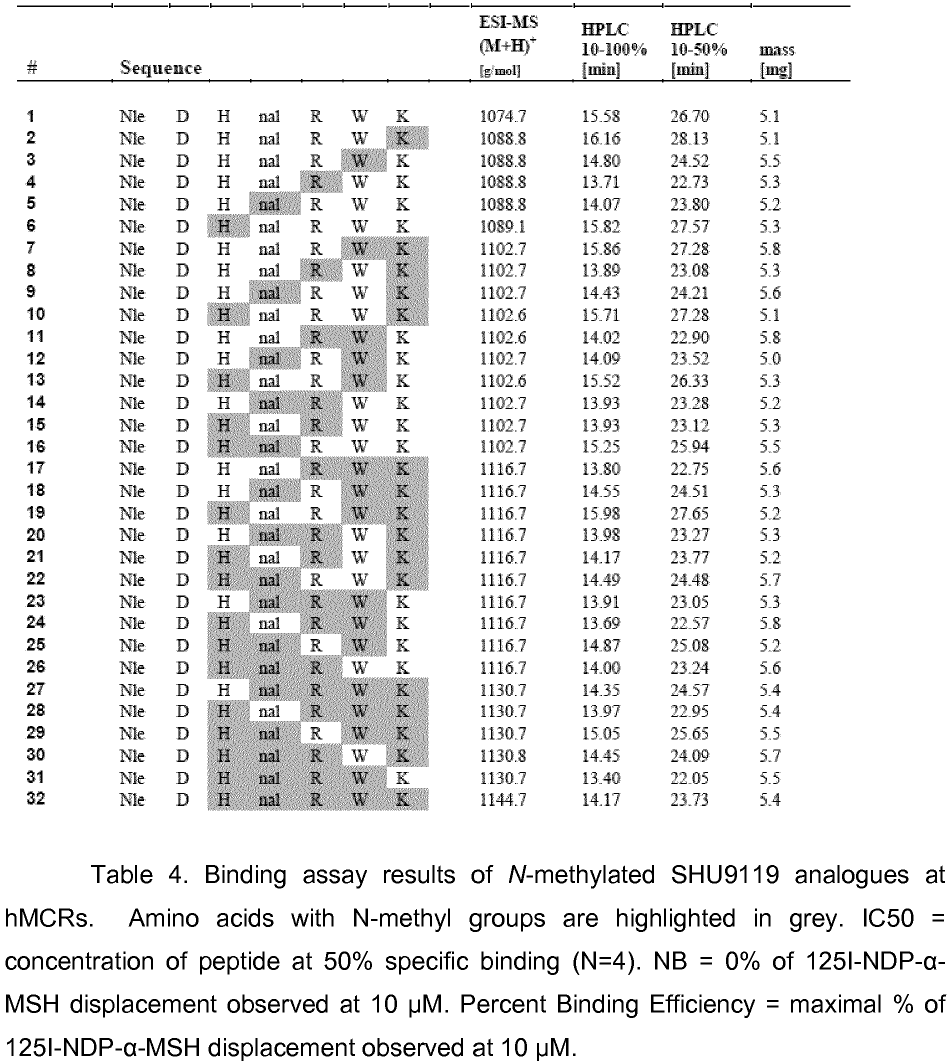

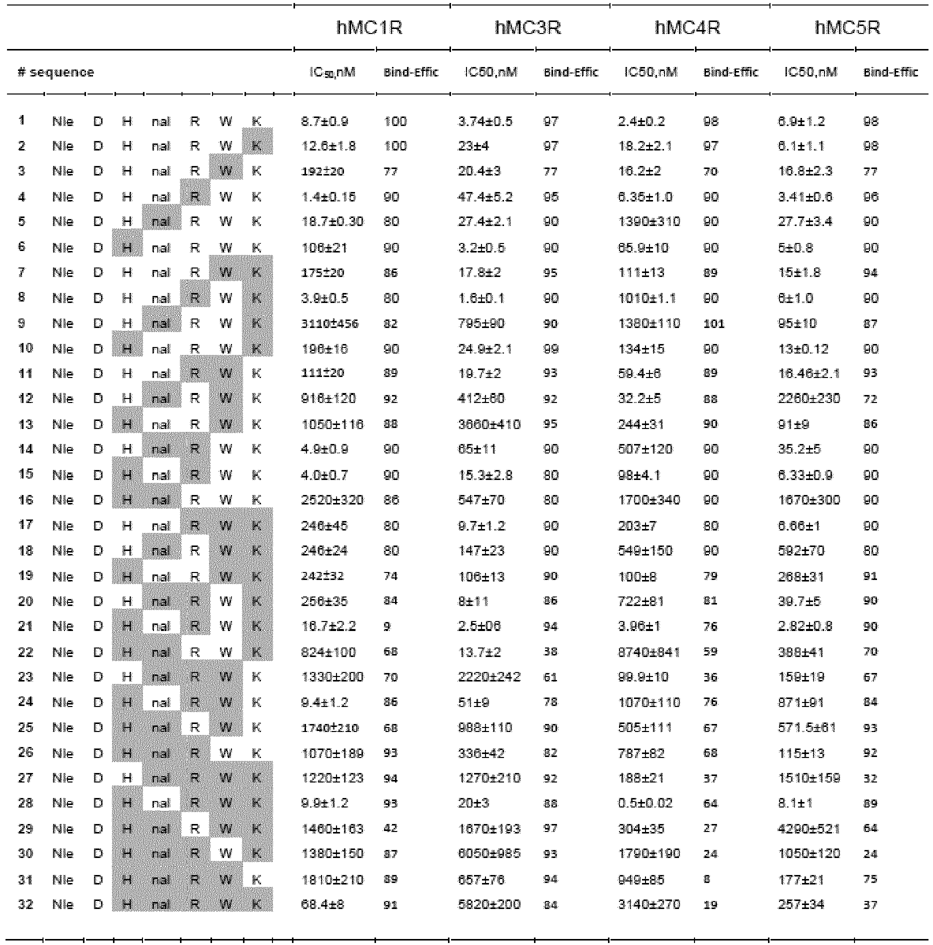

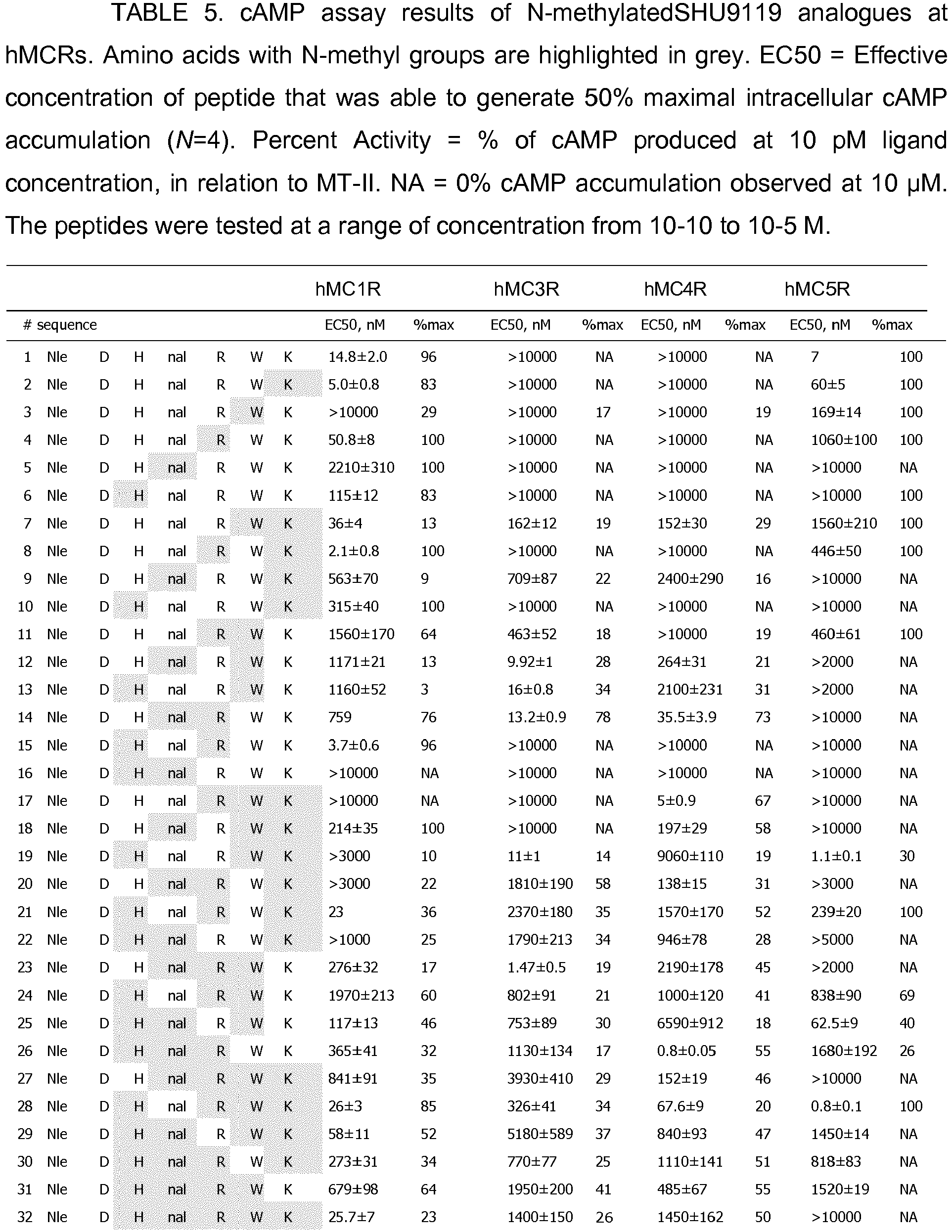

**Table 6: Chemical shift assignment of 15 in DMSO-d₆ at 300 K.**

| Residue | NH/*N*Me | Hα | Hβ | Hχ | Hδ | Hε/NHε | Hz/Others |
|---|---|---|---|---|---|---|---|
| N1e⁴ (norleucine) | 7.869 *J*_{NH-Hα}=8.2 Hz | 4.211 *J*_{NH-Hα}=8.2 Hz *J*_{Hα-Hβpro-S} =4.7 Hz *J*_{Hα-Hβpro-R} 9.3 Hz | 1 473 (*pro-S*) 1.373 *(pro-R) J*_{Hα-Hβpro-S} =4.7 Hz *J*_{Hα-Hβpro-R} =9.3 Hz | 1.252 | 1.252 | 0.854 (Hε) | 1.886 (protons of N-terminus COMe) |
| Asp⁵ (norleucine) | 8.296 *J*_{NH-Hα}=7.0 Hz | 4.835 *J*_{NH-Hα}=7.0 Hz *J*_{Hα-Hβpro-R} =4.9 Hz *J*_{Hα-Hβpro-S} =10.2 Hz | 2.797 (*pro-S*) 2.534 *(pro-R) J*_{Hα-Hβpro-R} =4.9 Hz *J*_{Hα-Hβpro-S} =10.2 Hz *J* _{Hβpro-R-Hβpro-S} =14.8 Hz | | | | |
| *N*Me-His⁶ | 2.978 | 5.355 *J*_{Hα-Hβ} =4.5 Hz *J*_{Hα-Hβ'} =11.1 Hz | 3.199 (β) 2.920 (β') *J*_{Hα-Hβ} =4.5 Hz *J*_{Hα-Hβ'} =11.1 Hz *J*_{Hβ-Hβ'} = 16.1 Hz | | | | |
| nal⁷ (D-Naphthyl Alanine) | 8.438 *J*_{NH-Hα}=8.3 Hz | 5 001 *J*_{NH-Hα}=8.3 Hz *J*_{Hα-Hβpro-R} =5.7 Hz *J*_{Hα-Hβpro-S} =9.0 Hz | 3.140 *(pro-R)* 3.044 (*pro-S*) *J*_{Hα-Hβpro-R} =5.7 Hz *J*_{Hα-Hβpro-S} =9.0 Hz *J* _{Hβpro-R-Hβpro-S} =14.1 Hz | 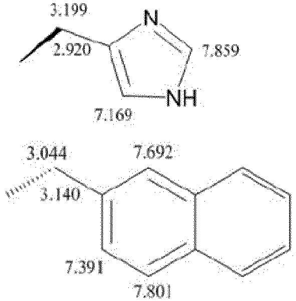 | | | |
| | | | | | | | |
| *N*Me-Arg⁸ | 2.868 | 4.305 (broad) | 1.881 (β) 1.660 (β') | 1.273 1.181 | 2.960 J_{Hδ-Hε} = 5.7 Hz | 7.749 J_{Hδ-Hε} = 5.7 Hz | |
| Trp⁹ | 7.602 *J*_{NH-Hα}=7.4 Hz | 4.427 *J*_{NH-Hα} =7.4 Hz *J*_{Hα-Hβ} =4.5 Hz *J*_{Hα-Hβ'} =8.7 Hz | 3.345 (β) 3.033 (β') *J*_{Hα-Hβ}=4.5 Hz *J*_{Hα-Hβ'}=8.7 Hz *J*_{Hβ-Hβ'} =14.8 Hz | | 7.139 *J*_{Hδ-Hε} =2.5 Hz | 10.822 *J*_{Hδ-Hε} =2.5Hz | 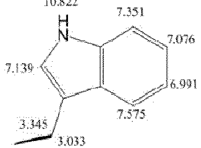 |
| Lys¹⁰ | 7.616 *J*_{NH-Hα}=7.4 Hz | 4.071 *J*_{NH-Hα}=7.4 Hz *J*_{Hα-Hβ} =4.0 Hz *J*_{Hα-Hβ'} =9.5 Hz | 1.694 (T) 1.694 (13') *J*_{Hα-Hβ} =4.50Hz *J*_{Hα-Hβ'} =9.5 Hz | 1.386 1.281 | 1.476 | 3.317 (ε) 3.107 (ε') *J*_{Hz-Hε} =5.7 Hz *J*_{HZ-Hε'} =5.7 Hz | 8.353 *J*_{Hz-Hε} =5.7 Hz *J*_{Hz-Hε'} =5.7 Hz 7.188 & 6.977 (protons of CONH₂protection) |

**Table 7: Chemical shift assignment of 17 in DMSO-d₆ at 300 K.**

| Residue | NH/*N*Me | Hα | Hβ | Hγ | Hδ | Hε/NHε | Hz/Others |
|---|---|---|---|---|---|---|---|
| Nle⁴ (norleucine) | 7.897 *J*_{NH-Hα}=8.2 Hz | 4.301 *J*_{NH-Hα}=8.2 Hz *J*_{Hα-Hβ*pro*-S}=5.0 Hz *J*_{Hα-Hβ*pro*-R}=8.6 Hz | 1.629 (*pro-*S) 1.449 *(pro-R) J*_{Hα-Hβ*pro*-S}=5.0 Hz *J*_{Hα-Hβ*pro*-R}=8.6 Hz *J*_{Hβ p*ro*-R-Hβ *pro*-S} =13.7 Hz | 1.260 | 1.260 | 0.869 (Hε) | 1.870 (protons of N-terminus COMe) |
| Asp⁵ | 8.020 *J*_{NH-Hα}=6.9 Hz | 4.545 *J*_{NH-Hα}=6.9 Hz *J*_{Hα-Hβ*pro*-R}=9.1 Hz *J*_{Hα-Hβ*pro*-S}=5.1 Hz | 2.463 *(pro-R)* 2.352 (*pro-*S) *J*_{Hα-Hβ*pro*-R}=9.1 Hz *J*_{Hα-Hβ*pro*-S}=5.1 Hz *J*_{Hβ *pro*-R-Hβ *pro*-S} =158 Hz | | | | |
| His⁶ | 8.528 *J*_{NH-Hα}=8.2 Hz | 4.684 *J*_{NH-Hα}=8.2 Hz *J*_{Hα-Hβ*pro*-R}=8.6 Hz *J*_{Hα-Hβ*pro*-S}=5.6 Hz | 3.069 (*pro-*S) 2.920 (*pro*-R) *J*_{Hα-Hβ*pro*-R}=8.6 Hz *J*_{Hα-Hβ*pro*-S}=5.6 Hz *J*_{Hβ*pro*-R-Hβ *pro*-S} =15.7 Hz | 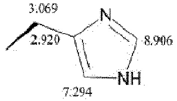 | | | |
| | | | | | | | |
| nal⁷ (D-Naphthyl Alanine) | 8.170 *J*_{NH-Hα}=8.2 Hz | 4.909 *J*_{NH-Hα}=8.2 Hz *J*_{Hα-Hβ*pro*-R}=5.6 Hz *J*_{Hα-Hβ*pro*-S}=8.7 Hz | 3.046 *(pro-R)* 2.925 (*pro-*S) *J*_{Hα-Hβ*pro*-R}=5.6 Hz *J*_{Hα-Hβpro-S}=8.7 Hz *J*_{Hβ *pro*-R-Hβ *pro*-S} =14.2 Hz | 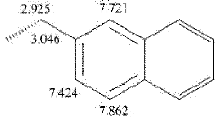 | | | |
| *N*Me-Arg⁸ | 1.989 | 5.238 *J*_{Hα-Hβ} =7.0 Hz *J*_{Hα-Hβ'} =7.0 Hz | 1.383 (β) 1.196 (β') *J*_{Hα-Hβ} =7.0 Hz *J*_{Hα-Hβ'} =7.0 Hz | 1.083 | 2.933 *J*_{Hδ-Hε} =6.0 Hz | 7.491 *J*_{Hδ-Hε} =6,0 Hz | |
| *N*Me-Trp⁹ | 2.677 | 5.829 *J*_{Hα-Hβ} =4.9 Hz *J*_{Hα-Hβ'} =9.8 Hz | 3.139 (β) 3.005 (β') *J*_{Hα-Hβ} =4.9 Hz *J*_{Hα-Hβ'}=9.8 Hz *J*_{Hβ-Hβ'} =14.8 Hz | | 7.193 *J*_{Hδ-Hε} =2.5 Hz | 10.766 *J*_{Hδ-Hε} =2.5 Hz | 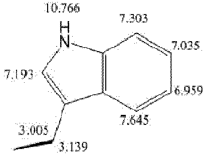 |
| *N*Me-Lys¹⁰ | 2.633 | 4.988 *J*_{Hα-Hβ} =12.3 Hz *J*_{Hα-Hβ'}=3.2 Hz | 1.896 (β) 1.644 (β') *J*_{Hα-Hβ} =12.3 Hz *J*_{Hα-Hβ'} =3.2 Hz | 1.462 1.329 | 1.220 1.005 | 3.294 (ε) 2.975 (ε') *J*_{Hz-Hε} =7.3 Hz *J*_{Hz-Hε'} =2.0 Hz | 7.885 *J*_{Hz-Hε} =7.3 Hz *J*_{Hz-Hε'}=2.0 Hz 7.1379 & 7.3456 (protons of CONH₂ protection) |

**Table 8: Temperature coefficient data of 15 and 17 in DMSO-d6. 1H-1D spectra were recorded for both the compounds at variable temperatures ranging from 295 K to 315 K in 5 K intervals and the temperature coefficients were calculated according to (Δδ^{∗}1000)/ΔT where in Δδ and ΔT are changes in chemical shift of the corresponding NH and the change in sample temperature, respectively.**

| Name of the Compound | | | |
|---|---|---|---|
| **15** | | **17** | |
| Name of the NH | Temperature Coefficient (ppb/K) | Name of the NH | Temperature Coefficient (ppb/K) |
| Nle⁴-NH | -3.50 | Nle⁴-NH | -4.00 |
| Asp⁵-NH | -6.50 | Asp⁵-NH | -6.00 |
| -- | - | His⁶-NH | -4.50 |
| Nal⁷-NH | -4.20 | Nal⁷-NH | -3.00 |
| Trp⁹-NH | -4.50 | -- | - |
| Lys¹⁰-NH | -3.00 | -- | - |
| Lys¹⁰-Hz | -5.40 | Lys¹⁰-Hz | -3.00 |

**Table 9: Backbone dihedral angles (in the cyclic core) measured from DG structures of 15 and 17.**

| Backbone Dihedral Angles in the order **15/17** | | | | | | | | | | | | |
|---|---|---|---|---|---|---|---|---|---|---|---|---|
| Dihedral Angle | | *N*Me-His⁶/His⁶ | | | Nal⁷ | | | *N*Me-Arg⁸ | | | Trp⁹/ *N*Me-Trp⁹ | |
| *φ* | | -119.5/-78.7 | | | 103.4/104.1 | | | -112.8/-107.4 | | | -90.3/-144.0 | |
| *ψ* | | 36.4/51.9 | | | -84.9/-155.0 | | | 28.4/88.9 | | | 6.5/51.7 | |

| Dihedral Angles for the SHU portion | | | | | | | | | | | | |
|---|---|---|---|---|---|---|---|---|---|---|---|---|
| SHU1/ *N*Me-SHU1 | SHU2/ *N*Me-SHU2 | | SHU3/ *N*Me-SHU3 | SHU4/ *N*Me-SHU4 | | SHU5/ *N*Me-SHU5 | SHU6/ *N*Me-SHU6 | | SHU7/ *N*Me-SHU7 | SHU8/ *N*Me-SHU8 | SHU9/ *N*Me-SHU9 | SHU10/ *N*Me-SHU10 |
| 167.9/ 63.8 | -50.3/ -99.4 | | 171.6/ 159.8 | 148.6/ 31.5 | | 3.2/ -95.0 | 83.7/ 175.5 | | 173.6/ 175,5 | 142.0/ -104.3 | 54.8/ 124.5 | -125.2/ -69.7 |

**Table 10: List of distance restraints that were used to derive the DG structure of 15 and the corresponding violations in rMD trajectory.**

| Residue | Atom | Residue | Atom | Upper Bound | Lower Bound | Mean distance (from MD) | Violation (A) |
|---|---|---|---|---|---|---|---|
| Nle | Hα | Asp | NH | 2,34 | 1,91 | 2,20 | |
| Nle | Hα | Asp | Hα | 4,82 | 3,94 | 4,27 | |
| Asp | Hα | *N*Me-His | Hα | 5,17 | 4,23 | 4,35 | |
| Asp | Hα | nal | NH | 4,35 | 3,56 | 3,99 | |
| Asp | Hα | Lys | Hz | 4,09 | 3,35 | 3,29 | |
| Asp | Hβ*pro-R* | Asp | NH | 2,75 | 2,25 | 2,45 | |
| Asp | Hβ*pro-R* | nal | NH | 4,7 | 3,85 | 4,84 | +0,1 |
| Asp | Hβpro-R | Lys | Hz | 2,75 | 2,2.5 | 2,20 | -0,05 |
| Asp | Hβpro-S | Asp | NH | 2,92 | 2,39 | 2,47 | |
| Asp | H*βpro-S* | nal | NH | 4,71 | 3,85 | 3,96 | |
| Asp | Hβ*pro-S* | Lys | Hz | 3,5 | 2,86 | 3,32 | |
| *N*Me-His | Hα | *N*Me-His | *N*Me | 4,85 | 3,64 | 3,72 | |
| *N*Me-His | Hα | nal | NH | 2,96 | 2,42 | 2,61 | |
| *N*Me-His | *N*Me | Asp | NH | 6,37 | 4,89 | 4,97 | |
| *N*Me-His | *N*Me | Asp | Hα | 3,15 | 2,25 | 2,68 | |
| *N*Me-His | *N*Me | nal | NH | 3,86 | 2,83 | 3,58 | |
| *N*Me-His | *N*Me | Lys | Hα | 5,66 | 4,3 | 3,76 | -0.6 |
| *N*Me-His | *N*Me | Lys | Hz | 5,98 | 4,56 | 5,16 | |
| nal | Hα | *N*Me-His | Hα | 5,34 | 437 | 4,12 | -0,2 |
| nal | Hα | nal | NH | 3,12 | 2,56 | 2,83 | |
| nal | Hα | Lys | NH | 5,23 | 4,28 | 5,25 | +0,02 |
| *N*M*e*-Arg | *N*Me | nal | NH | 5,11 | 3,85 | 5,12 | +0,01 |
| *N*M*e*-Arg | *N*Me | nal | Hα | 3,33 | 2,4 | 2,68 | |
| *N*M*e*-Arg | *N*Me | *N*M*e*-Arg | Hα | 5,13 | 3,87 | 3,68 | -0,2 |
| Lys | Hα | Asp | Hα | 5,92 | 4,84 | 4,92 | |
| Asp | Hα | Trp | hε3 (NH) | 5,17 | 4,23 | 6,03 | +0,9 |
| *N*Me-Arg | *N*Me | Trp | hδ1 | 5 | 3,76 | 4,25 | |
| *N*M*e*-Arg | *N*Me | Trp | hε1 | 6,19 | 4,74 | 5,42 | |

**Table 11: List of distance restraints that were used to derive the DG structure of 17 and the corresponding violations in fMD trajectory.**

| Residue | Atom | Residue | Atom | Upper Bound | Lower Bound | Mean distance (from MD) | Violation (A) |
|---|---|---|---|---|---|---|---|
| Nle | NH | Nle | Hα | 3,16 | 2,33 | 2,89 | |
| Nle | NH | Asp | Hα | 3,79 | 2,8 | 4,87 | +1,0 |
| Asp | NH | Nle | Hα | 2,38 | 1,76 | 2,50 | +0,1 |
| Asp | NH | Asp | Hα | 2,99 | 2,21 | 2,89 | |
| Asp | NH | His | Hα | 4,86 | 3,59 | 5,04 | +0,2 |
| Asp | Hα | *N*Me-Trp | *N*Me | 5,42 | 3,71 | 3,72 | |
| His | NH | Asp | NH | 5,25 | 3,88 | 4,14 | |
| His | NH | Asp | Hα | 2,35 | 1,74 | 2,30 | |
| His | NH | His | Hα | 2,98 | 2,2 1 | 2,88 | |
| His | NH | na1 | NH | 3,84 | 2,84 | 3,86 | +0,1 |
| His | NH | nal | Hα | 5,49 | 4,06 | 5,1 5 | |
| His | NH | *N*Me-Trp | *N*Me | 4,78 | 3,24 | 3,79 | |
| nal | NH | Asp | Hα | 4,44 | 3,28 | 5,03 | +0,6 |
| nal, | NH | Asp | Hβ*pro-S* | 4,41 | 3,26 | 4,11 | |
| nal | NH | His | Hα | 2,43 | 1,79 | 2,22 | |
| nal | NH | nal | Hα | 3,08 | 2,27 | 2,85 | |
| nal | NH | *N*M*e*-Arg | Hα | 5,25 | 3,88 | 4,74 | |
| nal | NH | *N*M*e*-Arg | *N*Me | 6,77 | 4,71 | 5,17 | |
| nal | Hα | *N*M*e*-Arg | *N*Me | 3,1 | 1.99 | 2,74 | |
| nal | hb2 | *N*M*e*-Arg | *N*Me | 4,18 | 2,79 | 3,47 | |
| nal | NH | *N*Me-Trp | *N*Me | 4,99 | 3,39 | 4,82 | |
| nal | Hα | *N*Me -Trp | *N*Me | 5,04 | 3,43 | 4,55 | |
| *N*M*e*-Arg | *N*Me | His | NH | 7,61 | 5,33 | 6,88 | |
| *N*Me-Arg | Hα | nal | Hα | 5,42 | 4,01 | 4,34 | |
| *N*M*e*-Arg | Hα | *N*M*e*-Arg | *N*Me | 4,74 | 3,21 | 3,73 | |
| *N*M*e*-Arg | Hα | *N*Me-Trp | *N*Me | 3,16 | 2,04 | 2,83 | |
| *N*M*e*-Arg | Hα | *N*Me-Lys | *N*Me | 3,44 | 2,25 | 4,38 | +1,0 |
| *N*Me-Trp | Hα | *N*M*e*-Arg | Hα | 5,03 | 3,72 | 4,26 | |
| *N*Me-Trp | Hα | *N*Me-Arg | *N*Me | 5,52 | 3,79 | 5,01 | |
| *N*Me-Trp | *N*Me | *N*Me-Arg | *N*Me | 5,78 | 3,68 | 4,63 | |
| *N*Me-Trp | Hα | *N*Me-Trp | *N*Me | 3,44 | 2,25 | 3,73 | +0,3 |
| *N*Me-Trp | Hα | *N*Me-Lys | Hα | 4,72 | 3,49 | 4,29 | |
| *N*Me-Trp | Hα | *N*Me-Lys | *N*Me | 3,19 | 2,06 | 2,82 | |
| *N*Me-Lys | Hz | Asp | Hβ*pro*-*R* | 2,72 | 2,01 | 2,46 | |
| *N*Me-Lys | Hz | *N*Me-Arg | Hα | 4,89 | 3,62 | 4,86 | |
| *N*Me-Lys | Hα | *N*Me-Trp | *N*Me | 4,46 | 3 | 4,85 | +0,4 |
| *N*Me-Lys | Hα | *N*Me-Lys | *N*Me | 4,32 | 2,9 | 3,72 | |

As used herein, the term "about" refers to plus or minus 10% of the referenced number.

The disclosures of the following U.S. Patents are incorporated in their entirety by reference herein: U.S. Pat. No. 14/117,949 and U.S. Pat. No. 5,674,839

Various modifications of the invention, in addition to those described herein, will be apparent to those skilled in the art from the foregoing description..

Although there has been shown and described the preferred embodiment of the present invention, it will be readily apparent to those skilled in the art that modifications may be made thereto which do not exceed the scope of the appended claims. Therefore, the scope of the invention is only to be limited by the following claims. Reference numbers recited in the claims are exemplary and for ease of review by the patent office only, and are not limiting in any way. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting of", and as such the written description requirement for claiming one or more embodiments of the present invention using the phrase "consisting of" is met.

### References

Gantz, I.; Fong, T. M., Am. J. Physiol. Endocrinol. Metab. 2003, 284, E468-E474.
Cone, R. D., Ed., The melanocortin system, Ann. N. Y. Acad. Sci. 2003, (994), 1-383.
Cone, R. D. Endocrine Rev. 2006, 27, 736-749.
Getting, S. J. Pharmacol. Ther. 2006, 111, 1-15.
Hadley, M. E.; Dorr, R. T. Peptides 2006, 27, 921-930.
Wikberg, J. E.; Mutulis, F. Nature Rev. Drug Disc. 2008, 7, 307-323.
Cai, M.; Nyberg, J.; Hruby, V. J. Curr. Top. Med. Chem. 2009, 9, 554-563.
Hadley, M. E., The Melanotropic Peptides, CRC Press: Boca Raton, Florida, 1988; Vols. I-III.
Cone, R. D., Ed., The Melanocortin Receptors, Humana Press: Totowa, New Jersey, 2000**.**
Brzoska T.; Böhm, M.; Lügering, A.; Loser, K.; Luger, T. A. Adv. Exp. Med. Biol. 2010, 681, 107-116.
Muceniece, R.; Dambrova, M. Adv. Exp. Med. Biol. 2010, 681, 61-70.
Wessells, H.; Fuciarelli, K.; Hansen, J.; Hadley, M. E.; Hruby, V. J.; Dorr, R.; Levine, N. J. Urol. 1998, 160, 389-393.
King, S. H.; Mayorov, A. V.; Balse-Srinivasan, P.; Hruby, V. J.; Vanderah, T W.; Wessells, H. Curr. Top. Med. Chem. 2007, 7, 1098-1106.
Ni, X. P., Butler, A. A.; Cone, R. D.; Humphreys, M. H. J. Hypertens. 2006, 24, 2239-2246.
Li, S. J.; Varga, K.; Archer, P.; Hruby, V. J.; Sharma, S. D.; Kesterson, R. A.; Cone, R. D.; Kunos, G. J. Neurosci. 1996, 16, 5182-5188.
Fan, W.; Boston, B. A.; Kesterson, R. A.; Hruby, V. J.; Cone, R. D. Nature 1997, 385, 165-168.
Vergoni, A. V.; Poggioloi, R.; Bertolini, A. Neuropeptides 1986, 7, 153-158.
Vergoni, A. V.; Poggioloi, R.; Marrama, D.; Bertolini, A. Eur. J. Pharmacol. 1990, 179, 347-355.
Ellacott, K. L. J.; Halatchev, I. G.; Cone, R. D. Peptides 2006, 27, 340-349.
Butler, A. A. Peptides 2006, 27, 281-290.
Yang, Y. K.; Harmon, C. M. Obes. Rev. 2003, 4, 239-248.
Morgan, C.; Thomas, R. E.; Cone, R. D. 2004, 45, 58-63.
Morgan, C.; Thomas, R. E.; Ma, W.; Novotny, M. V.; Cone, R. D. Chem. Senses. 2004, 29, 111-115.
Mogil, J. S.; Wilson, S. G.; Chesler, E. J.; Rankin, A. L.; Nemmani, K. V.; Lariviere, W. R.; Groce, M. K.; Wallace, M. R.; Kaplan, L.; Staud, R.; Ness, T. J.; Glover, T. L.; Stankova, M.; Mayorov, A.; Hruby, V. J.; Grisel, J. E.; Fillingim, R. B. Proc. Nat. Acad. Sci. U.S.A. 2003, 100, 4867-4872.
Kalange, A. S.; Kokare, D. M.; Singru, P. S.; Upadhya, M. A.; Chopde, C. T.; Subhedar, N. K. Brain Res. 2007, 1181, 10-20.
Vrinten, D. H.; Kalkman, C. J.; Adan, R. A.; Gispen, W. H. Eur. J. Pharmacol. 2001, 429, 61-69.
Van der Ploeg, L. H.; Martin, W. J.; Howard, A. D.; Nargund, R. P.; Austin, C. P.; Guan, X.; Drisko, J.; Cashen, D.; Sebhat, I.; Patchett, A. A.; Figueroa, D. J.; DiLella, A. G.; Connolly, B. M.; Weinberg, D. H.; Tan, C. P.; Palyha, O. C.; Pong, S. S.; MacNeil, T.; Rosenblum, C.; Vongs, A.; Tang, R.; Yu, H.; Sailer, A. W.; Fong, T. M.; Huang, C.; Tota, M. R.; Chang, R. S.; Stearns, R.; Tamvakopoulos, C.; Christ, G.; Drazen, D. L.; Spar, B. D.; Nelson, R. J.; MacIntyre, D. E. Proc. Nat. Acad. Sci. U.S.A. 2002, 99, 11381-11386.
Ellacott, K. L. J.; Cone, R. D. Recent Prog. Horm. Res. 2004, 59, 395-408.
Marks, D. L.; Hruby, V. J.; Brookhart, G.; Cone, R. D. Peptides 2006, 27, 259-264.
Cowley, M. A.; Smart, J. L.; Rubinstein, M.; Cerdán, M. G.; Diano, S.; Horvath, T. L.; Cone, R. D.; Low, M. J. Nature 2001, 411, 480-484.
Chen, W.; Kelly, M. A.; Opitz Araya, X.; Thomas, R. E.; Low, M. J.; Cone, R. D., Cell 1997, 91, 789-798.
Yang, Y. Eur. J. Pharmacol. 2011, 660, 125-130.
Cooray, S. N.; Clark, A. J. Mol. Cell. Endocrinol. 2011, 331, 215-221.
Hruby, V. J.; Wilkes, B. C.; Hadley, M. E.; Alobeidi, F.; Sawyer, T. K.; Staples, D. J.; Devaux, A. E.; Dym, O.; Castrucci, A. M. D.; Hintz, M. F.; Riehm, J. P.; Rao, K. R. J. Med. Chem. 1987, 30, 2126-2130.
Castrucci, A. M. L.; Hadley, M. E.; Sawyer, T. K.; Wilkes, B. C.; Al-Obeidi, F.; Staples, D. J.; Devaux, A. E.; Dym, O.; Hintz, M. F.; Riehm, J. P.; Rao, K. R.; Hruby, V. J. Gen. Comp. Endocrinol. 1989, 73, 157-163.
Hruby, V. J.; Cai, M.; Cain, J. P.; Mayorov, A. V.; Dedek, M. M.; Trivedi, D. Curr. Top. Med. Chem. 2007, 7, 1107-1119.
Fung, S.; Hruby, V. J. Curr. Opin. Chem. Biol. 2005, 9, 352-358.
Hruby, V. J.; Cai, M.; Grieco, P.; Han, G.; Kavarana, M.; Trivedi, D. Ann. N. Y. Acad. Sci. 2003, 994, 12-20.
Cai, M.; Mayorov, A. V.; Ying, J.; Stankova, M.; Trivedi, D.; Cabello, C.; Hruby, V. J. Peptides 2005, 26, 1481-1485.
Sawyer, T. K.; Sanfilippo, P. J.; Hruby, V. J.; Engel, M. H.; Heward, C. B.; Burnett, J. B.; Hadley, M. E. Proc. Nat. Acad. Sci. U.S.A. 1980, 77, 5754-5758.
Grieco, P.; Balse, P. M.; Weinberg, D.; MacNeil, T.; Hruby, V. J. J. Med. Chem. 2000, 43, 4998-5002.
Cai, M.; Mayorov, A. V.; Cabello, C.; Stankova, M.; Trivedi, D.; Hruby, V. J. J. Med. Chem. 2005, 48, 1839-1848.
Balse-Srinivasan, P.; Grieco, P.; Cai, M.; Trivedi, D.; Hruby, V. J. J. Med. Chem. 2003, 46, 3728-3733.
Han, G.; Haskell-Luevano, C.; Kendall, L.; Bonner, G.; Hadley, M. E.; Cone, R. D.; Hruby, V. J. J. Med. Chem. 2004, 47, 1514-1526.
Sawyer, T. K.; Hruby, V. J.; Darman, P. S.; Hadley, M. E. Proc. Nat. Acad. Sci. USA 1982, 79, 1751-1755.
Al-Obeidi, F.; Hadley, M. E.; Pettitt, B. M.; Hruby, V. J. J. Am. Chem. Soc. 1989, 111, 3413-3416.
Cai, M.; Cai, C.; Mayorov, A. V.; Xiong, C.; Cabello, C. M.; Soloshonok, V. A.; Swift, J. R.; Trivedi, D.; Hruby, V. J. J. Pept. Res. 2004, 63, 116-131.
Hruby, V.J., Lu, D.; Sharma, S.D. ; Castrucci, A.L.; Kesterson, R.A.; Al-Obeidi, F.A.; Hadley, M.E.; Cone, R.D. J. Med. Chem, 1995, 38, 3454-3461.
Grieco, P.; Cai, M.; Liu, L.; Mayorov, A.; Chandler, K.; Trivedi, D.; Lin, G.; Campiglia, P.; Novellino, E.; Hruby, V. J. J. Med. Chem. 2008, 51, 2701-2707.
Mayorov, A. V.; Han, S. Y.; Cai, M.; Hammer, M. R.; Trivedi, D.; Hruby, V. J. Chem. Biol. Drug. Des. 2006, 67, 329-335.
Mayorov, A. V.; Cai, M.; Palmer, E. S.; Dedek, M. M.; Cain, J. P.; Van Scoy, A. R.; Tan, B.; Vagner, J.; Trivedi, D.; Hruby, V. J. J. Med. Chem. 2008, 51, 187-195.
Bednarek, M. A.; MacNeil, T.; Tang, R.; Fong, T. M.; Cabello, M. A.; Maroto, M.; Teran, A. J. Med. Chem. 2007, 50, 2520-2526.
Mayorov, A. V.; Cai, M.; Chandler, K. B.; Petrov, R. R.; Van Scoy, A. R.; Yu, Z.; Tanaka, D. K.; Trivedi, D.; Hruby, V. J. J. Med. Chem. 2006, 49, 1946-1952.
Ballet, S.; Mayorov, A. V.; Cai, M.; Tymecka, D.; Chandler, K. B.; Palmer, E. S.; Rompaey, K. V.; Misicka, A.; Tourwé, D.; Hruby, V. J. Bioorg. Med. Chem. Lett. 2007, 17, 2492-2498.
Cain, J. P.; Mayorov, A. V.; Cai, M.; Wang, H.; Tan, B.; Chandler, K.; Lee, Y.; Petrov, R. R.; Trivedi, D.; Hruby, V. J. Bioorg. Med. Chem. Lett. 2006, 16, 5462-5467.
Sebhat, I. K.; Martin, W. J.; Ye, Z.; Barakat, K.; Mosley, R. T.; Johnston, D. B.; Bakshi, R.; Palucki, B.; Weinberg, D. H.; MacNeil, T.; Kalyani, R. N.; Tang, R.; Stearns, R. A.; Miller, R. R.; Tamvakopoulos, C.; Strack, A. M.; McGowan, E.; Cashen, D. E.; Drisko, J. E.; Hom, G. J.; Howard, A. D.; MacIntyre, D. E.; Van der Ploeg, L. H.; Patchett, A. A.; Nargund, R. P. J. Med. Chem. 2002, 45, 4589-4593.
Mutulis, F.; Kreicberga, J.; Yahorava, S.; Mutule, I.; Borisova-Jan, L.; Yahorau, A.; Muceniece, R.; Azena, S.; Veiksina, S.; Petrovska, R.; Wikberg, J. E. Bioorg. Med. Chem. 2007, 15, 5787-5810.
Todorovic, A.; Haskell-Luevano, C. Peptides 2005, 26, 2026-2036.
Richardson, T. I.; Ornstein, P. L.; Briner, K.; Fisher, M. J.; Backer, R. T.; Biggers, C. K.; Clay, M. P.; Emmerson, P. J.; Hertel, L. W.; Hsiung, H. M.; Husain, S.; Kahl, S. D.; Lee, J. A.; Lindstrom, T. D.; Martinelli, M. J.; Mayer, J. P.; Mullaney, J. T.; O'Brien, T. P.; Pawlak, J. M.; Revell, K. D.; Shah, J.; Zgombick, J. M.; Herr, R. J.; Melekhov, A.; Sampson, P. B.; King, C. H. J. Med. Chem. 2004, 47, 744-755.
Doedens, L.; Opperer, F.; Cai, M.; Beck, J. G.; Dedek, M., Palmer, E.; Hruby, V. J.; Kessler, H. J. Am. Chem. Soc. 2010, 132, 8115-8128.
Ying, J.; Kövér, K. E.; Gu, X.; Han, G.; Trivedi, D. B.; Kavarana, M. J.; Hruby, V. J. Biopolymers 2003, 71, 696-716.
Grieco, P.; Brancaccio, D.; Novellino, E.; Hruby, V. J.; Carotenuto, A. Eur. J. Med. Chem. 2011, 46, 3721-3733.
Holder, J. R.; Haskell-Luevano, C. Med. Res. Rev. 2004, 24, 325-356. Review
Grieco, P.; Han, G.; Weinberg, D.; Van der Ploeg, L. H.; Hruby, V. J. Biochem. Biophys. Res. Commun. 2002, 292, 1075-1080.
Grieco, P.; Lavecchia, A.; Cai, M.; Trivedi, D.; Weinberg, D.; MacNeil, T.; Van der Ploeg, L. H.; Hruby, V. J. J. Med. Chem. 2002, 45, 5287-5294. Unnatural
Wang, W.; Cai, M.; Xiong, C.; Zhang, J.; Trivedi, D.; Hruby, V. J. Tetrahedron 2002, 58, 7365-7374.
Chatterjee, J.; Gilon, C.; Hoffman, A.; Kessler, H. Acc. Chem. Res. 2008, 41, 1331-1342.
Chatterjee, J.; Laufer, B.; Kessler, H. Nat. Protoc. 2012, 7, 432-444.
Chatterjee, J.; Rechenmacher, F.; Kessler, H. Angew. Chem. Int. Ed. Engl.. 2013, 52, 254-269.
Biron, E.; Chatterjee, J.; Ovadia, O.; Langenegger, D.; Brueggen, J.; Hoyer, D.; Schmid, H.; Jelinek, R.; Gilon, C.; Hoffman, A.; Kessler, H. Angew. Chem. Int. Ed. Engl.. 2008, 47, 2595-2599.
White, T. R.; Renzelman, C. M.; Rand, A. C.; Rezai, T.; McEwen, C. M.; Gelev, V. M.; Turner, R. A.; Linington, R. G.; Leung, S. S.; Kalgutkar, A. S.; Bauman, J. N.; Zhang, Y.; Liras, S.; Price, D. A.; Mathiowetz, A. M.; Jacobson, M. P.; Lokey, R. S. Nat. Chem. Biol. 2011, 7, 810-817.
Ovadia, O.; Greenberg, S.; Chatterjee, J.; Laufer, B.; Opperer, F.; Kessler, H.; Gilon, C.; Hoffman, A. Mol. Pharmaceutics 2011, 8, 479-487.
Beck, J. G.; Chatterjee, J.; Laufer, B.; Kiran, M. U.; Frank, A. O.; Neubauer, S.; Ovadia, O.; Greenberg, S.; Gilon, C.; Hoffman, A.; Kessler, H. J. Am. Chem. Soc. 2012, 134, 12125-12133.
Havel, T. F. Prog. Biophys. Mol. Biol. 1991, 56, 43-78.
Lindahl, E.; Hess, B.; van der Spoel, D. J. Mol. Model. 2001, 7, 306-317.
Van der Spoel, D.; Lindahl, E., Hess, B.; Groenhof, G.; Mark, A. E.; Berendsen, H. J. C. J. Comput. Chem. 2005, 26, 1701-1718.
Van der Spoel, D.; Lindahl, E.; Hess, B.; Van Buuren, A. R.; Apol, E.; Meulenhoff, P. J.; Tieleman, D. P.; Sijbers, A. L. T. M.; Feenstra, K. A.; Van Drunen, R.; Berendsen, H. J. C. GROMACS User Manual, version 4.0, 2005**.**
Cavanagh, J.; Fairbrother, W. J.; Palmer III, A. G.; Rance, M.; Skelton, N. J. Protein NMR Spectroscopy (Second Edition): Principles and Practice, Academic Press Inc., San Diego, 2006**.**
Ramachandran, G. N.; Ramakrishnan, C.; Sasisekharan, V. J. Mol. Biol. 1963, 7, 95-99.
Richardson, J. S. Adv. Protein Chem. 1981, 34, 167-339.
Chou, K. C. Anal. Biochem. 2000, 286, 1-16.
Nair, C. M. ; Vijayan, M. ; Venkatachalapathi, Y. V.; Balaram, P. ; J. Chem. Soc. Chem. Commun. 1979, 1183-1184.
Gibbs, A. C.; Bjorndahl, T. C.; Hodges, R. S.; Wishart, D. S. J. Am. Chem. Soc. 2002, 124, 1203-1213.
Kessler, H.; Griesinger, C.; Wagner, K. J. Am. Chem. Soc. 1987, 109, 6927-6933.
Chen, M.; Aprahamian, C. J.; Celik, A.; Georgeson, K. E.; Garvey, W. T.; Harmon, C. M.; Yang, Y. Biochemistry 2006, 45, 1128-1137.
Larkin, M. A.; Blackshields, G.; Brown, N. P.; Chenna, R.; McGettigan, P. A.; McWilliam, H.; Valentin, F.; Wallace, I. M.; Wilm, A.; Lopez, R.; Thompson, J. D.; Gibson, T. J.; Higgins, D. G. Bioinformatics. 2007, 23, 2947-2948.
Gouy, M.; Guindon, S.; Gascuel, O. Mol. Biol. Evol. 2010, 27, 221-224.
Eswar, N.; Marti-Renom, M. A.; Webb, B.; Madhusudhan, M. S.; Eramian, D.; Shen, M.; Pieper, U.; Sali, A. Curr. Protoc. Bioinforma. (Baxevanis, A. D., Petsko, G. A., Stein, L. D. & Stormo, G. D., John Wiley & Sons, Inc.) 2006, Supplement 15, 5.6.1-5.6.30.
Bolton, E.; Wang, Y.; Thiessen, P. A.; Bryant, S. H. Annual Reports in Computational Chemistry: Volume 4 (Elsevier: Oxford, UK), 2008, 217-240.
Hanwell, M. D.; Curtis, D. E.; Lonie, D. C.; Vandermeersch, T.; Zurek, E.; Hutchison, G. R. J. Cheminform. 2012, 4, 1-17.
Morris, G. M.; Huey, R.; Lindstrom, W.; Sanner, M. F.; Belew, R. K.; Goodsell, D. S.; Olson, A. J. J. Comput. Chem. 2009, 30, 2785-2791.
Seeliger, D. ; de Groot, B. L. J. Comput. Aided. Mol. Des. 2010, 24, 417-422.
Trott, O.; Olson, A. J. J. Comput. Chem. 2010, 31, 455-461.

## Claims

1. A modified melanocortin receptor modulator, the modulator comprising Ac-Nle-c[Asp-His-nal-Arg-Trp-Lys]-NH₂, wherein at least one amino acid within the c[Asp-His-nal-Arg-Trp-Lys] portion of Ac-Nle-c[Asp-His-nal-Arg-Trp-Lys]-NH₂ is N-methylated,
**characterized in that**
the modulator is:
Ac-Nle-c[Asp-His-nal-(*NMe*)Arg-Trp-(*NMe*)Lys]-NH₂ (SEQ. No. 8),
Ac-Nle-c[Asp-(*NMe*)His-nal-Arg-Trp-(*NMe*)Lys]-NH₂ (SEQ. No. 10),
Ac-Nle-c[Asp-(*NMe*)His-nal-(*NMe*)Arg-Trp-Lys]-NH₂ (SEQ. NO. 15), or
Ac-Nle-c[Asp-(*NMe*)His-nal-(*NMe*)Arg-(*NMe*)Trp-(*NMe*)Lys]-NH₂ (SEQ. No. 28,
for medical use in modulating a melanocortin 1 (hMC1) receptor for regulating skin pigmentation by bringing about a desired skin coloration.

2. A non-medical and non-surgical use of a modulator of a melanocortin receptor for regulating skin pigmentation of a mammal, wherein a modified melanocortin receptor modulator is administered in an effective amount sufficient to bring about a desired skin coloration, wherein the modulator comprises Ac-Nle-c[Asp-His-nal-Arg-Trp-Lys]-NH₂, wherein at least one amino acid within the c[Asp-His-nal-Arg-Trp-Lys] portion of Ac-Nle-c[Asp-His-nal-Arg-Trp-Lys]-NH₂ is N-methylated, and is Ac-Nle-c[Asp-His-nal-(*NMe*)Arg-Trp-(*NMe*)Lys]-NH₂ (SEQ. No. 8), Ac-Nle-c[Asp-(*NMe*)His-nal-Arg-Trp-(*NMe*)Lys]-NH₂ (SEQ. No. 10), Ac-Nle-c[Asp-(*NMe*)His-nal-(*NMe*)Arg-Trp-Lys]-NH₂ (SEQ. NO. 15), or Ac-Nle-c[Asp-(*NMe*)His-nal-(*NMe*)Arg-(*NMe*)Trp-(*NMe*)Lys]-NH₂ (SEQ. No. 28.

## Patentansprüche

1. Modifizierter Melanocortinrezeptormodulator, wobei der Modulator Ac-Nle-c[Asp-His-nal-Arg-Trp-Lys]-NH₂ umfasst, wobei mindestens eine Aminosäure innerhalb des c[Asp-His-nal-Arg-Trp-Lys]-Abschnittes von Ac-Nle-c[Asp-His-nal-Arg-Trp-Lys]-NH₂ N-methyliert ist,
**dadurch gekennzeichnet, dass**
der Modulator ist:
Ac-Nle-c[Asp-His-nal-(*NMe*)Arg-Trp-(*NMe*)Lys]-NH₂ (SEQ. Nr. 8),
Ac-Nle-c[Asp-(*NMe*)His-nal-Arg-Trp-(*NMe*)Lys]-NH₂ (SEQ. Nr. 10),
Ac-Nle-c[Asp-(*NMe*)His-nal-(*NMe*)Arg-Trp-Lys]-NH₂ (SEQ. Nr. 15) oder
Ac-Nle-c[Asp-(*NMe*)His-nal-(*NMe*)Arg-(*NMe*)Trp-(*NMe*)Lys]-NH₂ (SEQ. Nr. 28,
zur medizinischen Verwendung beim Modulieren eines Melanocortin 1 (hMC1) Rezeptors zum Regulieren der Hautpigmentierung durch Herbeiführen einer gewünschten Hautfärbung.

2. Nichtmedizinische und nichtchirurgische Verwendung eines Modulators eines Melanocortinrezeptors zum Regulieren der Hautpigmentierung eines Säugetieres, wobei ein modifizierter Melanocortinrezeptormodulator in einer wirksamen Menge verabreicht wird, die ausreicht, eine gewünschte Hautfärbung herbeizuführen, wobei der Modulator Ac-Nle-c[Asp-His-nal-Arg-Trp-Lys]-NH₂ umfasst, wobei mindestens eine Aminosäure innerhalb des c[Asp-His-nal-Arg-Trp-Lys]-Abschnittes von Ac-Nle-c[Asp-His-nal-Arg-Trp-Lys]-NH₂ N-methyliert ist, und der Modulator Ac-Nle-c[Asp-His-nal-(*NMe*)Arg-Trp-(*NMe*)Lys]-NH₂ (SEQ. Nr. 8), Ac-Nle-c[Asp-(*NMe*)His-nal-Arg-Trp-(*NMe*)Lys]-NH₂ (SEQ. Nr. 10), Ac-Nle-c[Asp-(*NMe*)His-nal-(*NMe*)Arg-Trp-Lys]-NH₂ (SEQ. Nr. 15) oder Ac-Nle-c[Asp-(*NMe*)His-nal-(*NMe*)Arg-(*NMe*)Trp-(*NMe*)Lys]-NH₂ (SEQ. Nr. 28 ist.

## Revendications

1. Modulateur de récepteur de mélanocortine modifié, le modulateur comprenant Ac-Nle-c[Asp-His-nal-Arg-Trp-Lys]-NH₂, où au moins un acide aminé dans la partie c[Asp-His-nal-Arg-Trp-Lys] d'Ac-Nle-c[Asp-His-nal-Arg-Trp-Lys]-NH₂ est N-méthylé,
le modulateur étant **caractérisé en ce qu'**il est :
Ac-Nle-c[Asp-His-nal-(*NMe*)Arg-Trp-(*NMe*)Lys]-NH₂ (SEQ ID NO : 8),
Ac-Nle-c[Asp-(*NMe*)His-nal-Arg-Trp-(NMe)Lys]NH₂ (SEQ ID NO : 10),
Ac-Nle-c[Asp-(*NMe*)His-nal-(*NMe*)Arg-Trp-Lys]-NH₂ (SEQ ID NO : 15), ou
Ac-Nle-c[Asp-(*NMe*)His-nal-(NMe)Arg-(NMe)Trp-(NMe)Lys]-NH₂ (SEQ ID NO : 28),
pour une utilisation médicale dans la modulation d'un récepteur de mélanocortine 1 (hMC1) pour réguler la pigmentation de la peau en conférant une coloration souhaitée de la peau.

2. Utilisation non médicale et non chirurgicale d'un modulateur d'un récepteur de mélanocortine pour réguler la pigmentation de la peau d'un mammifère, où un modulateur de récepteur de mélanocortine est administré en une quantité efficace suffisante pour conférer une coloration souhaitée de la peau, où le modulateur comprend Ac-Nle-c[Asp-His-nal-Arg-Trp-Lys]-NH₂, où au moins un acide aminé dans la partie c[Asp-His-nal-Arg-Trp-Lys] d'Ac-Nle-c[Asp-His-nal-Arg-Trp-Lys]-NH₂ est N-méthylé, et est :
Ac-Nle-c[Asp-His-nal-(*NMe*)Arg-Trp-(*NMe*)Lys]-NH₂ (SEQ ID NO : 8),
Ac-Nle-c[Asp-(NMe)His-nal-Arg-Trp-(NMe)Lys]NH₂ (SEQ ID NO : 10),
Ac-Nle-c[Asp-(*NMe*)His-nal-(*NMe*)Arg-Trp-Lys]-NH₂ (SEQ ID NO : 15), ou
Ac-Nle-c[Asp-(NMe)His-nal-(*NMe*)Arg-(NMe)Trp-(*NMe*)Lys]-NH₂ (SEQ ID NO : 28).
